# EUROPEAN PATENT APPLICATION

(11) **EP 4 726 729 A1**
(43) Date of publication of application: **15.04.2026**
(21) Application number: 24891266.9
(22) Date of filing: 01.11.2024
(51) Int. Cl.: G16H 20/00, G06Q 50/10

(54) **SERVICE OUTPUT SYSTEM**

(30) Priority: 17.11.2023 JP 2023195638
(71) Applicant: NISHIKAWA CO., LTD., Tokyo 103-0006 (JP)
(72) Inventor: IJIMA Shinichi, Tokyo 103-0006 (JP); TSUJIKO Naoki, Tokyo 103-0006 (JP)
(74) Representative: Viering, Jentschura & Partner mbB Patent- und Rechtsanwälte
(86) International application number: PCT/JP2024/039161
(87) International publication number: WO 2025/105227

(57) **Abstract**

A service output system according to an embodiment comprises: for each user, an identification information assigning unit that assigns identification information identifying a user; a user specifying unit that specifies the user identified by the identification information; a sleep state acquisition unit that acquires a sleep state of the user; a purchase information acquisition unit that acquires purchase information indicating a product purchased by the user; a service specifying unit that specifies a service recommended to the user from the purchase information and the sleep state; a service output unit that outputs the service toward the user; and a content providing unit that provides life improvement content improving a life of the user.

## Description

### Technical Field

The present disclosure relates to a service output system. This application claims priority based on Japanese Patent Application No. 2023-195638 filed on November 17, 2023, and incorporates all contents described in said Japanese application.

### Background Art

JP 2020-197823 A describes an information processing system that selects a product preferable for a user. This system includes a biological data acquisition means for acquiring biological data during sleep of a user when a specific product is used from among a plurality of products, and a selection means for selecting a recommended product to be recommended to the user from among the plurality of products using the biological data when the specific product is used.

### Citation List

### Patent Literature

Patent Literature 1: JP 2020-197823 A

### Summary of Invention

### Problem to be Solved by the Invention

In the aforementioned information processing system, a recommended product to be recommended to the user is selected using the biological data of the user. However, since the recommended product recommended to the user is not what the user selected, there is a possibility that the recommended product differs from a product according to the preference of the user. Therefore, in addition to considering the actual situation of the sleep of the user, a proposal of a service according to the preference of the user is required. In a system that proposes a service to a user, it is required to improve the life of the user.

An object of the present disclosure is to provide a service output system capable of proposing a service according to a preference of the user in addition to considering the actual situation of the sleep of the user, and capable of improving the life of the user.

### Means for Solving the Problem

A service output system according to the present disclosure includes: (1) for each user, an identification information assigning unit that assigns identification information identifying a user; a user specifying unit that specifies the user identified by the identification information; a sleep state acquisition unit that acquires a sleep state of the user; a purchase information acquisition unit that acquires purchase information indicating a product purchased by the user; a service specifying unit that specifies a service recommended to the user from the purchase information and the sleep state; a service output unit that outputs the service toward the user; and a content providing unit that provides life improvement content improving a life of the user.

In the service output system according to the present disclosure, purchase information indicating a product purchased by the user is acquired. Since the user often purchases a product based on their own preference, the preference of the user tends to be reflected in the purchase information. In this service output system, from the purchase information in which the preference of the user is reflected and the sleep state, the service specifying unit specifies a service recommended to the user, and the service output unit outputs the service toward the user. Therefore, by acquiring the sleep state, in addition to considering the actual situation of the sleep of the user, a service according to the preference of the user can be proposed. In the service output system according to the present disclosure, as the life improvement content, for example, information indicating an exercise for making sleep good can be provided toward the user. Therefore, the life of the user can be improved.
(2) In the above (1), the life improvement content may include audio content. The service output system may further comprise a reception unit that receives a selection of audio content by the user from a plurality of types of audio content set in advance. The content providing unit may provide the audio content selected by the user in the reception unit at at least one of a time of falling asleep and a time of awakening of the user. In this case, the user can enjoy the audio content selected by themselves at at least one of the time of falling asleep and the time of awakening. For example, when the user selects audio content suitable for falling asleep as the audio content provided at the time of falling asleep, the user can smoothly fall asleep by the audio content provided at the time of falling asleep. For example, when the user selects audio content suitable for awakening as the audio content provided at the time of awakening, the user becomes easy to awaken by the audio content provided at the time of awakening.
(3) In the above (1) or (2), the life improvement content may include audio content. The content providing unit may provide audio content according to the sleep state at at least one of the time of falling asleep and the time of awakening of the user. In this case, since the audio content according to the sleep state is provided to the user, it is possible to make it easy for the user to fall asleep or awaken.
(4) In any one of the above (1) to (3), the content providing unit may provide advice according to the sleep state of the user. In this case, since the user can receive advice according to their own sleep state, the user can make their own sleep good based on the advice.
(5) In any one of the above (1) to (4), the content providing unit may provide information prompting behavior modification according to the sleep state of the user. For example, when the sleep state of the user is not good, it is possible to prompt behavior modification of the user so as to consult a sleep specialist. Therefore, by prompting a change in usual behavior to the user, such as giving an incentive to consult a specialist to the user, the life of the user can be made good.
(6) In any one of the above (1) to (5), the service output system may further comprise a point assigning unit that assigns points according to the sleep state to the user. For example, in a case where larger points are assigned to the user as the sleep of the user state is better, it can be a motivation for the user to strive to take sleep. Therefore, the sleep of the user can be made good.
(7) In any one of the above (1) to (6), the service output system may further comprise a sleep state output unit that outputs the sleep state toward the user, and a device control unit that controls an operation of a device constituting an environment around the user from the sleep state. In this case, since the sleep state is output to the user, the user can grasp their own sleep state. Since the operation of the device of, for example, an air conditioner arranged around the user can be controlled according to the sleep of the user state, the sleep of the user environment can be made comfortable according to the sleep state.
(8) In the above (7), the sleep state output unit may output, as the sleep state, a state of an autonomic nerve of the user and a health risk due to an abnormal respiratory state of the user. In this case, the user can grasp the state of their own autonomic nerve and the health risk due to the abnormal respiratory state. Therefore, the user can grasp their own sleep state in more detail.
(9) In any one of the above (1) to (8), the service output system may further comprise a sharing unit that outputs the sleep state of the user toward another user different from the user. In this case, since the sleep state of the user is output toward the other user, the other user can grasp the sleep state of the user. Since the other user can grasp the sleep state of the user, the other user can promptly perform a countermeasure for the sleep state of the user as necessary.
(10) In any one of the above (1) to (9), the service output system may further comprise a recording unit that records sounds emitted in the user and around the user during sleep of the user. In this case, the user can confirm, for example, their own snoring which is difficult to be aware of, and sounds emitted in the sleep environment during sleep. By listening to the sounds emitted from the user, the user can grasp the state of their own body during sleep. Furthermore, by listening to the sounds emitted around the user, the user can grasp their own sleep environment. **In** this way, the user can grasp their own sleep state by listening to the sounds recorded in the recording unit.

### Effect of the Invention

According to the present disclosure, in addition to considering the actual situation of the sleep of the user, a service according to the preference of the user can be proposed, and the life of the user can be improved.

### Brief Description of Drawings

[FIG. 1] FIG. 1 is a diagram showing a service output system according to an embodiment.
[FIG. 2] FIG. 2 is a block diagram showing functional configurations of a sleep analysis application and a service proposal application shown in FIG. 1.
[FIG. 3] FIG. 3 is a diagram showing an example of an authentication screen.
[FIG. 4] FIG. 4 is a diagram showing an example of a sleep state output screen.
[FIG. 5] FIG. 5 is a diagram showing an example of an audio record display screen.
[FIG. 6] FIG. 6 is a diagram showing an example of a sleep state output screen and an example of a consultation office display screen.
[FIG. 7] FIG. 7 is a diagram showing an example of a sleep state output screen.
[FIG. 8] FIG. 8 is a diagram showing an example of a sleep state output screen.
[FIG. 9] FIG. 9 is a diagram showing an example of a sleep state output screen.
[FIG. 10] FIG. 10 is a diagram showing an example of a sleep sharing result screen.
[FIG. 11] FIG. 11 is a diagram showing an example of an alarm setting screen.
[FIG. 12] FIG. 12 is a diagram showing an example of an alarm content setting screen and an example of a talent voice setting screen.
[FIG. 13] FIG. 13 is a diagram showing an example of a content selection screen.
[FIG. 14] FIG. 14 is a diagram showing an example of a video content screen.
[FIG. 15] FIG. 15 is a diagram showing an example of a member information display screen.
[FIG. 16] FIG. 16 is a flowchart showing steps of a part of an operation of the service output system according to an embodiment.
[FIG. 17] FIG. 17 is a flowchart showing steps of a part of an operation of the service output system according to an embodiment.
[FIG. 18] FIG. 18 is a flowchart showing steps of a part of an operation of the service output system according to an embodiment.
[FIG. 19] FIG. 19 is a flowchart showing steps of a part of an operation of the service output system according to an embodiment.
[FIG. 20] FIG. 20 is a flowchart showing steps of a part of an operation of the service output system according to an embodiment.

### Description of Embodiments

Hereinafter, embodiments of a service output system according to the present disclosure will be described with reference to the drawings. In the description of the drawings, the same or corresponding elements are denoted by the same reference numerals, and overlapping descriptions are omitted as appropriate. The drawings may be partially simplified or exaggerated for ease of understanding, and dimensional ratios and the like are not limited to those described in the drawings.

The service output system according to the present disclosure outputs a service recommended to a user toward the user. The service output system may be used, for example, for personal use or home use. The service output system may be used in a hospital or the like for therapeutic use. The user is a person who uses the service output system. The user is, for example, a person to whom a service is output by the service output system. The user may be, for example, a person having trouble regarding sleep, or a person receiving sleep treatment at a hospital or the like. "Outputting a service toward a user" means providing a service to the user. "Outputting a service toward a user" indicates, for example, transmitting information of a recommended service to a terminal owned by the user. In this case, the user can improve their own life by viewing the service displayed on the display of their own terminal. "Output toward a user" indicates transmitting information to the user.

A service is labor or benefit provided to a user. Services include both paid ones and free ones. A service includes, for example, a proposal (an advertisement as an example) of a product recommended to the user. A product is goods for the purpose of buying and selling. Products include both tangible objects and intangible objects. A product that is a tangible object includes, for example, bedding. In the present embodiment, a product is included in a service.

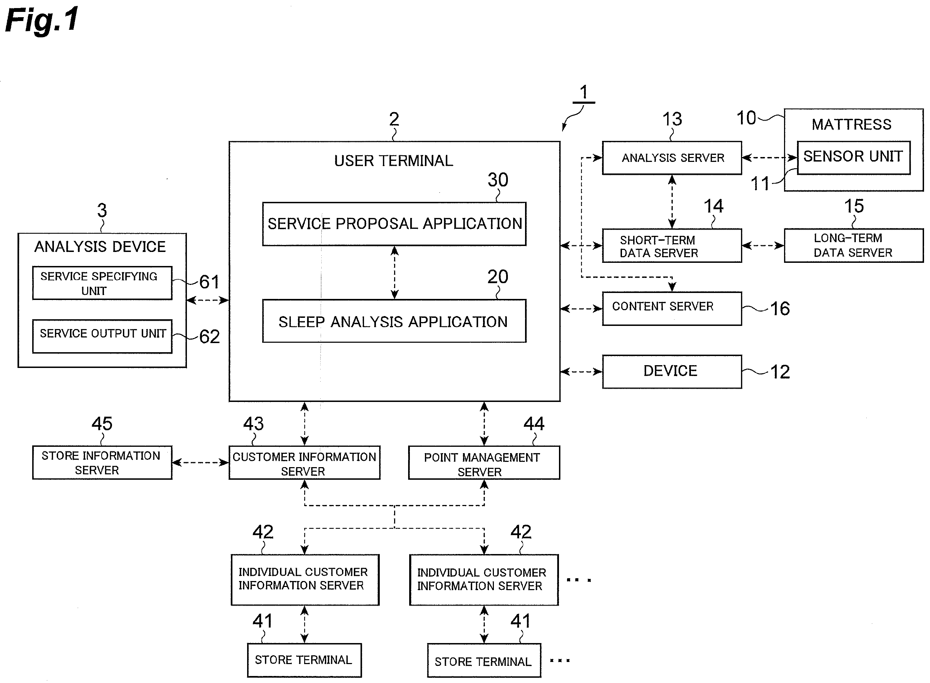

FIG. 1 is a diagram showing a service output system 1 according to an embodiment. The service output system 1 comprises a user terminal 2. The user terminal 2 is, for example, a mobile terminal. A mobile terminal is a portable information terminal. The mobile terminal is, for example, a mobile phone including a smartphone, a tablet, a laptop computer, or a wearable terminal. The user terminal 2 may be a terminal other than a mobile terminal, and may be, for example, a desktop computer. The user terminal 2 comprises, as an example, a processor (e.g., CPU) that executes an operating system and software (application), a main storage unit composed of ROM and RAM, an auxiliary storage unit composed of flash memory, a communication control unit composed of a wireless communication module, an input device, and an output device such as a display. However, the configuration of the user terminal 2 is not limited to the above and can be changed as appropriate.

In the user terminal 2, a sleep analysis application 20 and a service proposal application 30 are executed as application programs. Each of the sleep analysis application 20 and the service proposal application 30 (hereinafter, may be referred to as "application") may be downloaded to the user terminal 2 and executed in the user terminal 2, or may be executed in a device external to the user terminal 2 (e.g., a server). The application may be downloaded from the external device. Hereinafter, an example in which the application is downloaded to the user terminal 2 and the functions of the application are executed in the user terminal 2 will be described.

Each function of the application is realized by causing the processor or the main storage unit to read predetermined software and executing the software. The processor operates the aforementioned communication control unit, input device, or output device according to the software, and performs reading and writing of data in the main storage unit or the auxiliary storage unit. Data or a database necessary for execution of the functions of the application is stored in the main storage unit or the auxiliary storage unit.

Each functional element of the user terminal 2 is realized by causing the processor or a storage unit (e.g., the aforementioned main storage unit or auxiliary storage unit) to read predetermined software and executing the software. The processor operates the aforementioned communication control unit, input device, or output device according to the software, and performs reading and writing of data in the storage unit. Data or a database used for processing of the aforementioned external device is stored in the storage unit.

The application may be a distributed processing system executed by a plurality of computers, a client-server system, or a cloud system. The application according to the present embodiment includes, for example, a main module, a data acquisition module, a determination module, and an output module. Each functional element of the application functions by executing the data acquisition module, the determination module, and the output module. The application may be provided, as an example, after being fixedly recorded on a tangible storage medium such as a CD-ROM, a DVD-ROM, or a semiconductor memory. The application may be provided via a communication network as a data signal superimposed on a carrier wave. The functional configuration of the application will be described later.

The sleep analysis application 20 and the service proposal application 30 can share information with each other. "Sharing information" means that information can be provided from the sleep analysis application 20 to the service proposal application 30, and information can be provided from the service proposal application 30 to the sleep analysis application 20.

In the present embodiment, the service output system 1 comprises a sensor unit 11 and a device 12. The sensor unit 11 acquires, for example, at least one of respiration information, body movement information, and heart rate information during sleep of the user. However, the sensor unit 11 may acquire at least one of respiration information, body movement information, and heart rate information at a time other than during sleep of the user (e.g., during awakening). In the present embodiment, the sensor unit 11 acquires respiration information, body movement information, and heart rate information. "Acquiring" indicates performing measurement to obtain a measurement value, or receiving data and storing the data in the storage unit. The respiration information is information indicating the respiration of the user. The body movement information is information indicating the body of the user movement. The heart rate information is information indicating the heart rate of the user. The content of information acquired by the sensor unit 11 can be changed as appropriate. The sensor unit 11 may acquire, for example, at least one of information indicating the body of the user temperature, information indicating the blood pressure of the user, and information indicating the blood glucose level of the user.

In the present embodiment, the sensor unit 11 is attached to a mattress 10. The sensor unit 11 is, for example, a sensor sheet. However, the sensor unit 11 may be attached to a cushion. In this case, the sensor unit 11 may be a cushion sensor attached to the cushion. The sensor unit 11 may be, for example, an activity meter attached to the body of the user.

The sensor unit 11 has, for example, a sheet-like fabric on which a thread-like sensor is embroidered. The thread-like sensor is fixed to the sheet-like fabric by embroidery so as to spread two-dimensionally in the sheet-like fabric, for example. The thread-like sensor is, for example, a piezoelectric sensor. In this case, the piezoelectric sensor of the sensor sheet generates an electrical signal according to a load from the applied load of the body of the user. The sensor sheet acquires the electrical signal as the aforementioned respiration information, body movement information, and heart rate information. However, the type of sensor of the sensor sheet is not limited to the piezoelectric sensor and is not particularly limited. For example, the sensor sheet may include an acceleration sensor.

The sensor unit 11 outputs the respiration information, body movement information, and heart rate information as electrical signals generated by the above thread-like sensor (e.g., piezoelectric sensor) to the outside of the sensor unit 11. In the present embodiment, the sensor unit 11 transmits the respiration information, body movement information, and heart rate information to an analysis server 13 (described later).

The device 12 constitutes an environment around the user. "Constituting an environment around the user" includes, for example, that the device 12 affects at least one of temperature, brightness, sound, and scent around the user by operating. The device 12 is, for example, an air conditioner, a lighting fixture, a music player, or an aroma diffuser installed in the bedroom of the user. In the present embodiment, the device 12 is capable of communicating with the user terminal 2. The device 12 receives information for controlling the operation of the device 12 from the user terminal 2. The device 12 controls its own operation from the received information. However, the device 12 may be capable of communicating with the user terminal 2 via a content server 16 (described later). In this case, the content server 16 receives information for controlling the operation of the device 12 from the user terminal 2. The content server 16 may control the operation of the device 12 from the received information.

In the present embodiment, the service output system 1 further comprises an analysis server 13, a short-term data server 14, a long-term data server 15, and a content server 16.

The analysis server 13 analyzes the sleep of the user. The analysis server 13 acquires respiration information, body movement information, and heart rate information from the sensor unit 11. The analysis server 13 acquires a sleep state, which is a state of the sleep of the user, from the acquired respiration information, body movement information, and heart rate information. The sleep state includes, for example, at least one of a sleep stage, a sleep time, a sleep onset latency (time to fall asleep), a number of mid-sleep awakenings, sleep efficiency, a state of an autonomic nerve, and a health risk due to an abnormal respiratory state. The sleep state may include information other than the state of sleep, such as a fatigue level and a stress level, in addition to the state of the sleep of the user. In the present embodiment, the sleep state includes the sleep stage, the sleep time, the sleep onset latency (time to fall asleep), the number of mid-sleep awakenings, the sleep efficiency, the state of the autonomic nerve, and the health risk due to the abnormal respiratory state.

An example of processing in which the analysis server 13 acquires the sleep stage will be described. The sleep stage is an index indicating the depth of the sleep of the user. The sleep stage is classified into, for example, awakening, REM sleep, and non-REM sleep. Non-REM sleep is classified into light sleep and deep sleep. Note that non-REM sleep may be classified into three or more types of sleep stages. The analysis server 13 acquires the sleep of the user stage from, for example, the respiration information, body movement information, and heart rate information acquired from the sensor unit 11.

An example of processing in which the analysis server 13 acquires the sleep time and the sleep onset latency will be described. The analysis server 13 acquires the bedtime and wake-up time of the user. The bedtime is the time when the user went to bed. The wake-up time is the time when the user woke up the next morning or the like. The analysis server 13 determines and acquires the bedtime and the wake-up time from, for example, the body movement information acquired by the sensor unit 11 and the movement of the mattress 10 detected by the sensor unit 11 (e.g., acceleration sensor). The analysis server 13 acquires the sleep of the user onset time and awakening time from the acquired bedtime, wake-up time, and sleep stage. The analysis server 13 acquires, for example, a time when the sleep of the user stage changed from an awakening stage to a REM sleep stage or a non-REM sleep stage between the bedtime and the wake-up time as the sleep onset time. The analysis server 13 acquires, for example, a time when the sleep of the user stage finally became the awakening stage between the bedtime and the wake-up time as the awakening time.

The analysis server 13 acquires the sleep time from, for example, the acquired sleep onset time and awakening time. The analysis server 13 acquires the time from the sleep onset time to the awakening time as the sleep time. The analysis server 13 acquires the sleep onset latency from, for example, the acquired bedtime and sleep onset time. The analysis server 13 acquires, for example, the time from the bedtime to the sleep onset time as the sleep onset latency.

An example of processing in which the analysis server 13 acquires the number of mid-sleep awakenings will be described. Mid-sleep awakening is that the user awakens between the sleep onset time and the awakening time. "Awakening" here means that the sleep stage changes from the REM sleep stage or the non-REM sleep stage to the awakening stage. The analysis server 13 acquires the number of mid-sleep awakenings from, for example, the acquired sleep onset time, awakening time, and sleep stage. The analysis server 13 acquires the number of times the sleep stage changed from the REM sleep stage or the non-REM sleep stage to the awakening stage between the sleep onset time and the awakening time as the number of mid-sleep awakenings.

An example of processing in which the analysis server 13 acquires the sleep efficiency will be described. Sleep efficiency is an index for evaluating the quality of sleep. The analysis server 13 acquires a mid-sleep awakening time from, for example, the acquired sleep onset time, awakening time, and sleep stage. The mid-sleep awakening time is, for example, a time from a time when the user had a mid-sleep awakening to a time when the sleep of the user stage next became the REM sleep or non-REM sleep stage. Note that when the mid-sleep awakening time is acquired multiple times, the mid-sleep awakening times are summed up.

The analysis server 13 acquires the sleep efficiency from, for example, the acquired bedtime, sleep onset time, awakening time, and mid-sleep awakening time. The analysis server 13 acquires, for example, a value obtained by dividing a time obtained by subtracting the mid-sleep awakening time from the time from the sleep onset time to the awakening time by the time from the bedtime to the sleep onset time as the sleep efficiency.

An example of processing in which the analysis server 13 acquires the state of the autonomic nerve will be described. The autonomic nerve is a nerve that controls the function of organs such as the heart or stomach, or involuntary functions such as blood circulation. Involuntary indicates, for example, not being as one wishes or not being according to the intention. The autonomic nerve consists of the sympathetic nerve and the parasympathetic nerve. The sympathetic nerve and the parasympathetic nerve are adjusted while balancing each other.

The analysis server 13 acquires the heart rate of the user variability (HRV) by analyzing the heart rate information acquired by, for example, the sensor unit 11. **In** the present embodiment, the analysis server 13 acquires a periodic component included in the heart rate variability from the heart rate of the user variability. The analysis server 13 performs frequency analysis on the periodic component of the heart rate variability and acquires a power spectrum of each frequency.

The power spectrum of the autonomic nerve obtained as a result of the above frequency analysis is divided into an LF (Low Frequency) component which is an integral value of the power spectrum in a low frequency band (0.04 Hz to 0.15 Hz as an example) and an HF (High Frequency) component which is an integral value of the power spectrum in a high frequency band (0.15 Hz to 0.4 Hz as an example). The LF component reflects sympathetic nerve activity and parasympathetic nerve activity, and the HF component reflects parasympathetic nerve activity.

The analysis server 13 acquires a sympathetic nerve index and a parasympathetic nerve index. The sympathetic nerve index is an index indicating the dominance of the sympathetic nerve. The sympathetic nerve index is, as an example, a value obtained by dividing the value of the LF component by the value of the HF component. The parasympathetic nerve index is an index indicating the dominance of the parasympathetic nerve. The parasympathetic nerve index is, as an example, a value obtained by dividing the value of the HF component by the sum of the LF component and the HF component.

The analysis server 13 acquires an active level during awakening from the acquired sympathetic nerve index. The active level is a degree indicating the dominance of the sympathetic nerve over the parasympathetic nerve. The analysis server 13 acquires a relaxation level during falling asleep from the acquired parasympathetic nerve index. The relaxation level is a degree indicating the dominance of the parasympathetic nerve over the sympathetic nerve. The analysis server 13 acquires the acquired relaxation level during falling asleep and active level during awakening as the state of the autonomic nerve.

An example of processing in which the analysis server 13 acquires the health risk due to the abnormal respiratory state will be described. The health risk is, for example, a risk of heart failure or the like associated with Sleep Apnea Syndrome (SAS). The abnormal respiratory state is a state of respiration during sleep that may pose a health risk to the user. The abnormal respiratory state includes, for example, an apnea state and a hypopnea state. The apnea state is a state in which the respiration of the user stops for 10 seconds or more. The hypopnea state is a state in which the amplitude of airflow in the respiration of the user decreases by 30% or more, and a state in which arterial oxygen saturation (SpO₂) decreases by 4% or more elapses for 10 seconds or more.

The analysis server 13 detects the abnormal respiratory state of the user. The analysis server 13 detects the abnormal respiratory state from at least one of the respiration information, body movement information, and heart rate information acquired by the sensor unit 11. The analysis server 13 detects the abnormal respiratory state from, for example, the respiratory rate of the user.

The analysis server 13 acquires the number of detections of the detected abnormal respiratory state for each unit time zone between the sleep onset time and the awakening time. The unit time zone is a time zone having a predetermined time width. The time width of the unit time zone is, for example, 1 hour. For example, the time width of the unit time zone can be changed as appropriate. As the time width of the unit time zone is smaller, it becomes possible to examine the sleep of the user in more detail, and the health risk can be determined with higher accuracy. On the other hand, as the time width of the unit time zone is larger, the processing amount of the analysis server 13 can be reduced.

The analysis server 13 acquires an average number of detections from the acquired number of detections of the abnormal respiratory state. The average number of detections is a value obtained by dividing the total value of the number of detections of the abnormal respiratory state between the sleep onset time and the awakening time by the time from the sleep onset time to the awakening time. When the time width of the unit time zone is 1 hour, the average number of detections corresponds to the so-called AHI (Apnea Hypopnea Index).

The analysis server 13 acquires a maximum number of detections from the acquired number of detections of the abnormal respiratory state. The maximum number of detections is the maximum value among the numbers of detections of the abnormal respiratory state acquired for each unit time zone. For example, the analysis server 13 acquires the number of detections of the abnormal respiratory state for each unit time zone. The analysis server 13 acquires the number of detections of the abnormal respiratory state in the unit time zone having the largest number of detections as the maximum number of detections.

The analysis server 13 acquires the health risk of the user. The analysis server 13 acquires, for example, the acquired average number of detections and maximum number of detections as the health risk due to the abnormal respiratory state.

The analysis server 13 outputs the acquired sleep state to the short-term data server 14 and the content server 16.

The short-term data server 14 stores the sleep state acquired from the analysis server 13. The short-term data server 14 stores the sleep of the user state in the most recent first period. The first period is, for example, 1 month or more and 5 months or less (3 months as an example). The short-term data server 14 outputs, for example, the sleep state for a period exceeding the first period to the long-term data server 15. The short-term data server 14 outputs the stored sleep state to the user terminal 2, for example, when receiving an output command described later from the user terminal 2.

The long-term data server 15 stores the sleep state acquired from the short-term data server 14. The long-term data server 15 stores the sleep of the user state in a second period longer than the aforementioned first period. The second period is, for example, 1 year or more and 3 years or less (2 years as an example).

The sleep state stored in the long-term data server 15 may be provided to the outside of the service output system 1. For example, the sleep state stored in the long-term data server 15 may be provided to a company where the user works. In this case, the company can manage the sleep of the user state. The sleep state stored in the long-term data server 15 may be provided to, for example, a medical institution such as a hospital. In this case, the medical institution can make use of the sleep state for the treatment of the user. The long-term data server 15 may store a result of a questionnaire on sleep for the user in addition to the sleep state. The questionnaire result may be provided to the outside of the service output system 1 (e.g., a medical institution) together with the sleep state.

The content server 16 stores life improvement contents for improving the life of the user in advance. The life improvement content is content for prompting improvement of the life of the user. The life improvement content includes video content, image content, and text content. In the present embodiment, the life improvement content includes audio content. The audio content includes music content and voice content. More specifically, the audio content includes at least one of a voice of a person, music such as healing music, an alarm sound, and a natural sound such as a wave sound.

The life improvement content includes, in addition to the audio content, for example, a video prompting an exercise for making the sleep of the user good. The exercise is, for example, yoga and stretching. By receiving the video prompting the exercise from the content server 16 as audio content, the user can acquire information serving as a trigger for making sleep good. The content server 16 is capable of communicating with the user terminal 2.

The life improvement content includes advice regarding the sleep of the user. The advice includes, for example, at least one of advice to the effect that there is a tendency to have difficulty falling asleep, and advice to the effect that continuous sleep is not taken. The advice includes, for example, advice for improving the sleep of the user. The advice for improving sleep includes, for example, behavior during awakening effective for reducing the number of mid-sleep awakenings.

The life improvement content includes information prompting behavior modification of the user. Behavior modification is that the user changes their own behavior. The information prompting behavior modification includes, for example, information prompting to go to a place where consultation regarding sleep is possible (hereinafter, may be referred to as "sleep consultation office") and consult a sleep specialist. The sleep consultation office includes, for example, a hospital specializing in sleep, a hospital or clinic having knowledge regarding sleep, and a counseling room other than a hospital. The sleep specialist may be, for example, a doctor specializing in sleep, or a person other than a doctor. The information prompting behavior modification includes, for example, information indicating a position of a sleep consultation office set in advance.

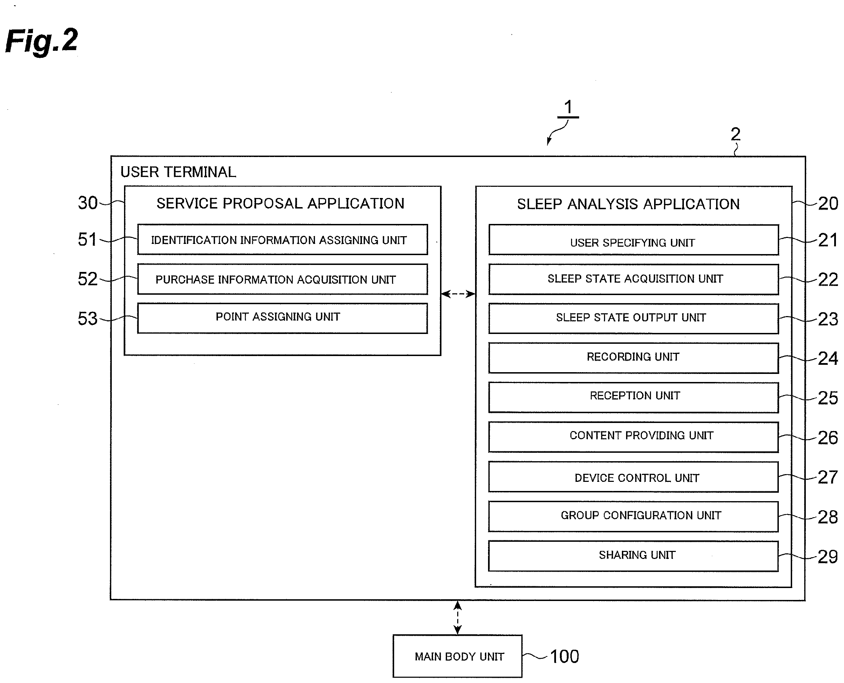

The functional configuration of the sleep analysis application 20 will be described. As shown in FIG. 2, the sleep analysis application 20 has, as its functional configuration, a user specifying unit 21, a sleep state acquisition unit 22, a sleep state output unit 23, a recording unit 24, a reception unit 25, a content providing unit 26, a device control unit 27, a group configuration unit 28, and a sharing unit 29.

The user specifying unit 21 specifies a user identified by identification information. The identification information is information for identifying a user. In the present embodiment, the identification information is assigned in advance by an identification information assigning unit 51 described later. The identification information is, for example, an ID for uniquely specifying a user. The user specifying unit 21 receives, for example, an input of the identification information and a password linked to the identification information by the user. The password is set in advance in association with the identification information, for example.

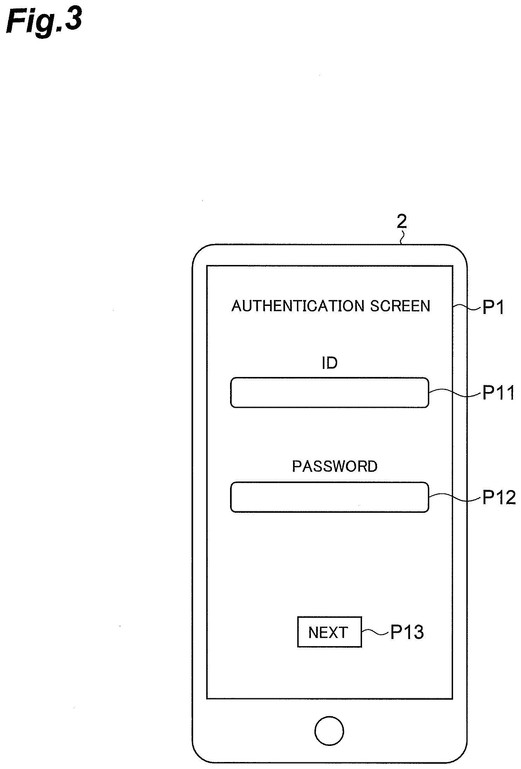

FIG. 3 is a diagram showing an example of an authentication screen P1. The user terminal 2 displays the authentication screen P1. The user terminal 2 displays the authentication screen P1 on the display of the user terminal 2 according to instructions of a program constituting the sleep analysis application 20. When the user terminal 2 is a smartphone, the display unit is, for example, a display of the smartphone.

The authentication screen P1 is a screen for performing authentication of a user. Performing authentication of a user is specifying a user identified by identification information. The authentication screen P1 includes an identification information input unit P11, a password input unit P12, and a determination unit P13. The identification information input unit P11 is a part where identification information is input by the user. The password input unit P12 is a part where a password is input by the user. The determination unit P13 is a part pressed after the identification information is input to the identification information input unit P11 and the password is input to the password input unit P12.

When the determination unit P13 is pressed, the user specifying unit 21 determines whether or not the identification information assigned by the identification information assigning unit 51 described later matches the identification information input to the identification information input unit P11. When the determination unit P13 is pressed, the user specifying unit 21 determines whether or not the password set in advance in association with the identification information by the user matches the password input to the password input unit P12. When it is determined that the assigned identification information and the input identification information match, and it is determined that the preset password and the input password match, the user specifying unit 21 specifies the user who input the identification information and the password as the user identified by the identification information.

When the user specifying unit 21 specifies the user identified by the identification information, the sleep analysis application 20 and the service proposal application 30 enter a state capable of sharing information with each other. Note that before the user specifying unit 21 specifies the user, the sleep analysis application 20 and the service proposal application 30 operate independently of each other. Before the user specifying unit 21 specifies the user, the sleep analysis application 20 acquires the sleep state acquired by the analysis server 13 and executes predetermined processing using the acquired sleep state. Before the user specifying unit 21 specifies the user, the service proposal application 30 executes predetermined processing independently of the processing executed by the sleep analysis application 20.

The sleep state acquisition unit 22 acquires the sleep state acquired by the analysis server 13. As a more specific example, the sleep state acquisition unit 22 transmits an output command to the short-term data server 14 at a predetermined timing. The predetermined timing is, for example, a preset time. The output command is a command for causing the short-term data server 14 to transmit the sleep state to the user terminal 2. The sleep state acquisition unit 22 acquires, as the sleep state, the sleep stage, the sleep time, the sleep onset latency, the number of mid-sleep awakenings, the sleep efficiency, the state of the autonomic nerve, and the health risk due to the abnormal respiratory state.

In the present embodiment, the sleep state acquisition unit 22 acquires an action record of the user. The action record is information indicating the presence or absence of an action of the user affecting sleep. The action of the user affecting sleep includes, for example, at least one of intake of caffeine, intake of alcohol, exercise, and napping. The sleep state acquisition unit 22 receives, for example, an input of the action record by the user and acquires the received action record. The sleep state acquisition unit 22 acquires a post-awakening evaluation of the user. The post-awakening evaluation is a subjective evaluation of sleep after awakening of the user. The subjective evaluation of sleep is an evaluation by the subjectivity regarding their own sleep of the user, such as having slept well today or not having slept very well today. The sleep state acquisition unit 22 receives, for example, an input of the post-awakening evaluation by the user after awakening and acquires the received post-awakening evaluation. The user inputs the post-awakening evaluation in multiple stages (5 stages as an example), for example.

The sleep state output unit 23 transmits the sleep state acquired by the sleep state acquisition unit 22 to the user terminal 2. The sleep state output unit 23 outputs a sleep score according to the sleep state acquired by the sleep state acquisition unit 22. The sleep score is an index for evaluating the quality of the sleep of the user. The sleep state output unit 23 calculates the sleep score from the sleep time, the sleep onset latency, the number of mid-sleep awakenings, and the sleep efficiency acquired by the sleep state acquisition unit 22. The sleep state output unit 23 normalizes each of the sleep time, the sleep onset latency, the number of mid-sleep awakenings, and the sleep efficiency, for example. The sleep state output unit 23 may calculate a total value of respective values of the normalized sleep time, sleep onset latency, number of mid-sleep awakenings, and sleep efficiency as the sleep score. In the present embodiment, the sleep state output unit 23 shares the sleep state acquired by the sleep state acquisition unit 22 with the service proposal application 30.

The recording unit 24 records sounds emitted in the user and around the user during sleep of the user. The sound emitted in the user is a sound emitted from the user, and includes, for example, the snoring of the user. The sound emitted around the user includes, for example, an operation sound of a device, and an environmental sound inside and outside the bedroom of the user. The recording unit 24 records sounds emitted in the user and around the user via, for example, a microphone mounted on the user terminal 2 to generate an audio record. In the present embodiment, the recording unit 24 generates an audio record for each day. The recording unit 24 may have, for example, a noise canceling function.

The reception unit 25 receives a selection of audio content by the user from a plurality of types of audio content set in advance. The user selects audio content by operating the user terminal 2, for example.

The content providing unit 26 provides life improvement content. "Providing life improvement content" indicates, for example, transmitting life improvement content or a link to life improvement content to the user terminal 2 so that the user can execute the life improvement content by operating the user terminal 2. **In** the present embodiment, the content providing unit 26 provides advice according to the sleep of the user state. The content providing unit 26 provides advice according to the sleep state acquired by the sleep state acquisition unit 22 to the user. The content providing unit 26 acquires advice regarding the sleep of the user from the content server 16.

The content providing unit 26 determines, for example, whether or not the sleep onset latency acquired by the sleep state acquisition unit 22 is equal to or longer than a predetermined time. When determining that the sleep onset latency is equal to or longer than the predetermined time, for example, the content providing unit 26 receives advice to the effect that there is a tendency to have difficulty falling asleep among advice regarding sleep from the content server 16. The content providing unit 26 determines, for example, whether or not the number of mid-sleep awakenings acquired by the sleep state acquisition unit 22 is equal to or greater than a predetermined number. For example, when determining that the number of mid-sleep awakenings is equal to or greater than the predetermined number, the content providing unit 26 receives advice to the effect that continuous sleep is not taken among advice regarding sleep from the content server 16.

When determining that the number of mid-sleep awakenings is equal to or greater than the predetermined number, for example, the content providing unit 26 receives behavior during awakening effective for reducing the number of mid-sleep awakenings among advice regarding sleep from the content server 16. "Behavior during awakening effective for reducing the number of mid-sleep awakenings" indicates, for example, exercise that is desirable to perform to reduce stress when the number of mid-sleep awakenings is large due to stress, and indicates refraining from alcohol the next day when mid-sleep awakenings are frequent due to excessive intake of alcohol, etc.

The content providing unit 26 provides the received advice regarding sleep, for example. "Providing advice" indicates, for example, displaying the advice on the display of the user terminal 2 so that the user can view the advice.

The content providing unit 26 provides information prompting behavior modification according to the sleep of the user state. The content providing unit 26 acquires, for example, the sleep score calculated by the sleep state output unit 23. The content providing unit 26 determines, for example, whether or not the acquired sleep score is equal to or less than a predetermined value. When determining that the acquired sleep score is equal to or less than the predetermined value, for example, the content providing unit 26 receives information prompting to consult a sleep specialist among information prompting behavior modification from the content server 16.

The content providing unit 26 provides the received information prompting behavior modification. "Providing information" indicates displaying the information on the display of the user terminal 2 so that the user can view the information.

In the present embodiment, the content providing unit 26 provides life improvement content according to the audio record generated by the recording unit 24. The content providing unit 26 acquires, for example, the volume, length, and frequency of the snoring of the user from the audio record generated by the recording unit 24. The content providing unit 26 calculates the severity of snoring from the acquired volume, length, and frequency of snoring. The severity of snoring is a degree indicating the height of health risk determined from the content of snoring.

The content providing unit 26 provides life improvement content according to the calculated severity of snoring. The content providing unit 26 determines, for example, whether or not the calculated severity of snoring is equal to or greater than a predetermined value. When determining that the calculated severity of snoring is equal to or greater than the predetermined value, the content providing unit 26 may provide, for example, information prompting to consult a sleep specialist as information prompting behavior modification. When determining that the calculated severity of snoring is equal to or greater than the predetermined value, the content providing unit 26 provides, for example, information indicating behavior during awakening effective for improving snoring. The information includes, for example, information prompting dietary restriction and exercise effective for eliminating obesity that can be a cause of snoring.

When the content providing unit 26 provides life improvement content according to the audio record, the user can receive life improvement content according to, for example, their own snoring which is difficult to be aware of. For example, when the severity of the snoring of the user is equal to or greater than a predetermined value, information prompting behavior modification can be provided to the user as life improvement content. **In** this way, since life improvement content can be provided in consideration of sounds emitted during the sleep of the user, the life of the user can be improved more effectively.

**In** the present embodiment, the content providing unit 26 provides audio content according to the sleep state acquired by the sleep state acquisition unit 22. "Providing audio content" includes, for example, making the audio content reproducible in the user terminal 2. The content providing unit 26 provides audio content according to the sleep state at at least one of the time of falling asleep and the time of awakening of the user.

The content providing unit 26 adjusts the volume of the audio content according to the sleep state, for example. When the audio content is a voice of a person, the content providing unit 26 changes the content of the voice of a person according to the sleep state, for example. The content of the voice of a person includes, for example, at least one of the lines and tone of voice of the person.

The content providing unit 26 acquires, for example, the sleep score calculated by the sleep state output unit 23. The content providing unit 26 determines whether or not the acquired sleep score is equal to or greater than a predetermined value. When determining that the sleep score is equal to or greater than the predetermined value, the content providing unit 26 sets the volume of the audio content to a predetermined first volume, for example. When the audio content is a voice of a person, the content providing unit 26 reproduces, for example, a voice with gentle content as the audio content. The voice with gentle content is, for example, a voice with gentle lines and tone.

When determining that the sleep score is not equal to or greater than the predetermined value, the content providing unit 26 sets the volume of the audio content to a predetermined second volume larger than the aforementioned first volume, for example. When the audio content is a voice of a person, the content providing unit 26 reproduces, for example, a voice with content gently calling attention to the user as the audio content. The voice with content gently calling attention is, for example, a voice calling attention with a gentle tone so that the user takes good sleep.

The aforementioned person is, for example, selectable by the user in advance in the reception unit 25. The aforementioned voice with gentle content and voice with content gently calling attention can be set in advance, for example. For example, every time a predetermined period (1 year as an example) elapses, the content of a new voice of a person may be downloaded to the user terminal 2. The download may be performed automatically every time the predetermined period (e.g., 1 year) elapses, or may be performed manually by the user.

The content providing unit 26 provides the audio content selected by the user in the reception unit 25. The content providing unit 26 provides the audio content at at least one of the time of falling asleep and the time of awakening of the user. In the present embodiment, the content providing unit 26 provides the audio content at the time of falling asleep and the time of awakening of the user. The content providing unit 26 reproduces the audio content at the time of falling asleep of the user by receiving an operation of the user terminal 2 by the user immediately before falling asleep, for example. The content providing unit 26 reproduces the audio content at the time of awakening of the user by reproducing the audio content at a reproduction time preset by the user, for example.

The device control unit 27 controls the operation of the device 12 from the sleep state. The device control unit 27 is capable of communicating with the device 12. For example, when the device 12 is an air conditioner, the device control unit 27 controls the temperature of the space where the user is present by controlling the operation of the device 12. For example, when the device 12 is a lighting fixture, the device control unit 27 controls the brightness of the space where the user is present by controlling the operation of the device 12. For example, when the device 12 is a music player, the device control unit 27 controls at least one of the type and volume of sound emitted in the space where the user is present by controlling the operation of the device 12. For example, when the device 12 is an aroma diffuser, the device control unit 27 controls the scent of the space where the user is present by controlling the operation of the device 12.

In the present embodiment, the device control unit 27 controls the operation of the device 12 from the audio record generated by the recording unit 24. The device control unit 27 acquires, for example, the volume, length, and frequency of the snoring of the user from the audio record generated by the recording unit 24. The device control unit 27 calculates the severity of snoring from the acquired volume, length, and frequency of snoring. The device control unit 27 determines whether or not the calculated severity of snoring is equal to or greater than a predetermined value.

When determining that the severity of snoring is equal to or greater than the predetermined value, the device control unit 27 makes the sleep of the user environment an environment easy to relax by controlling the operation of the device 12, for example. "Making the sleep of the user environment an environment easy to relax" indicates, for example, adjusting the sleep environment to an environment that the user feels comfortable, such as setting the temperature of the bedroom of the user to a temperature suitable for the sleep of the user, setting the lighting of the bedroom of the user to brightness suitable for sleep, or generating a scent suitable for sleep in the bedroom of the user. For example, when the device 12 is a music player, the device control unit 27 controls the operation of the music player to generate a sound suitable for sleep. The sound suitable for sleep is, for example, healing music.

The group configuration unit 28 generates a group composed of a plurality of users. In the present embodiment, the group configuration unit 28 generates a group composed of a plurality of users to whom identification information has been assigned by the identification information assigning unit 51. The group configuration unit 28 generates a group composed of a plurality of users arbitrarily determined by at least one user. The group includes, for example, a user and the family of the user.

The sharing unit 29 outputs the sleep state of the user toward another user different from the user. In the present embodiment, the sharing unit 29 outputs the sleep state of one user included in the group generated by the group configuration unit 28 to another user included in the group. For example, the sharing unit 29 makes it possible for the other user to grasp the sleep state of the one user by transmitting the sleep state of the one user to a user terminal owned by the other user.

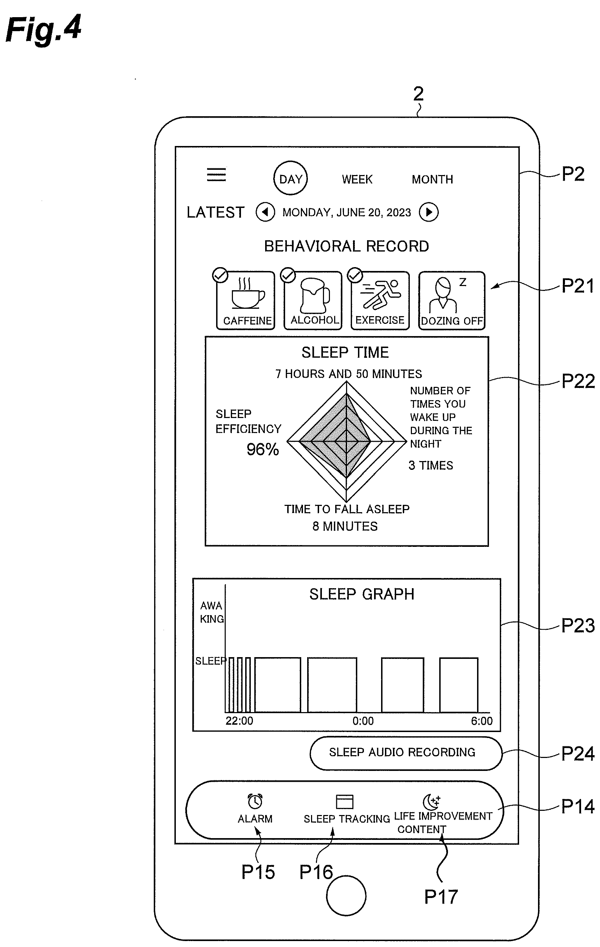

FIG. 4 is a diagram showing an example of a sleep state output screen P2. As shown in Figs. 3 and 4, when the determination unit P13 is pressed on the authentication screen P1, the user terminal 2 displays the sleep state output screen P2. The sleep state output screen P2 includes a menu bar P14. The menu bar P14 includes an alarm selection unit P15, a sleep record selection unit P16, and a content selection unit P17.

For example, the menu bar P14 is displayed before the sleep state output screen P2 is displayed. In this state, when the sleep record selection unit P16 is pressed, the user terminal 2 displays the sleep state output screen P2. The sleep state output screen P2 displays each of the sleep state for each day, the sleep state for each week, and the sleep state for each month. Examples of screens when the alarm selection unit P15 and the content selection unit P17 are pressed will be described later.

The sleep state output screen P2 displays, for example, the sleep state for each day. The sleep state output screen P2 includes an action record display unit P21, a radar chart display unit P22, a sleep graph display unit P23, and an audio record display unit P24.

The action record display unit P21 is a part that displays an action record input by the user. The action record display unit P21 includes, for example, at least one of an icon indicating that the user ingested caffeine, an icon indicating that the user drank alcohol, an icon indicating that the user performed exercise, and an icon indicating that the user took a nap. The content of the action record display unit P21 can be changed as appropriate. The sleep state acquisition unit 22 receives, for example, an action record input on another screen (not shown) different from the sleep state output screen P2. The sleep state acquisition unit 22 causes the action record display unit P21 to display the received action record. The sleep state acquisition unit 22 attaches a check mark to an icon corresponding to the action taken by the user from the received action record, for example.

As described above, the sleep state acquisition unit 22 causes the display of the user terminal 2 to display the action record of the user as the action record display unit P21. By the sleep state acquisition unit 22 displaying the action record in this way, the user can confirm their own action record. For example, the user can grasp the relationship between their own action and the sleep state by comparing their own action record and the sleep state. As a result, the user can obtain a trigger to review their own action, and the life of the user can be improved.

The radar chart display unit P22 is a part that displays the sleep time, sleep onset latency (time to fall asleep), number of mid-sleep awakenings, and sleep efficiency acquired by the sleep state acquisition unit 22. The radar chart display unit P22 displays, for example, a polygonal (e.g., quadrangular) radar chart. The radar chart display unit P22 may display a pentagonal radar chart. The sleep state output unit 23 causes the radar chart display unit P22 to display the sleep time, sleep onset latency, number of mid-sleep awakenings, and sleep efficiency acquired by the sleep state acquisition unit 22 as a radar chart.

The sleep graph display unit P23 is a part that displays a sleep graph. The sleep graph is a graph showing a change in sleep stage over time. The horizontal axis of the sleep graph indicates time, for example. The vertical axis of the sleep graph indicates the sleep stage, for example. In the present embodiment, the sleep graph display unit P23 displays the sleep of the user stage in two stages of sleep and awakening.

The sleep state output unit 23 causes the sleep graph display unit P23 to display a time zone in which the sleep stage acquired by the sleep state acquisition unit 22 is the REM sleep or non-REM sleep stage as sleep. The sleep state output unit 23 causes the sleep graph display unit P23 to display a time zone in which the sleep stage acquired by the sleep state acquisition unit 22 is the awakening stage as awakening. The content of the sleep graph display unit P23 can be changed as appropriate. For example, the sleep graph display unit P23 may display the sleep of the user stage in three stages of awakening, REM sleep, and non-REM sleep.

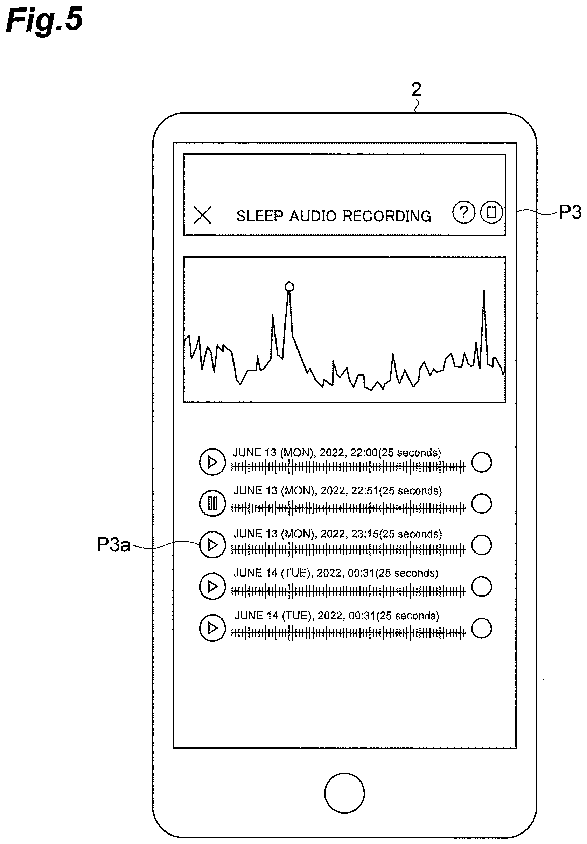

The audio record display unit P24 is a part for instructing display of an audio record. When the audio record display unit P24 is pressed, the user terminal 2 displays an audio record display screen P3. FIG. 5 is a diagram showing an example of the audio record display screen P3. The audio record display screen P3 displays the audio record generated by the recording unit 24. The audio record display screen P3 includes, for example, a graph showing the magnitude of sounds emitted in the user and around the user.

The recording unit 24 generates audio data indicating sound in a time zone in which a sound having a volume equal to or greater than a predetermined threshold occurred from the generated audio record. The time width of the time zone is, for example, 5 seconds or more and 1 minute or less (25 seconds as an example). The recording unit 24 determines whether or not there is a time point when a sound having a volume equal to or greater than the predetermined threshold occurred in the generated audio record. When determining that there is a time point when a sound having a volume equal to or greater than the predetermined threshold occurred, the recording unit 24 generates data indicating sound in a time zone including the time point as audio data. The recording unit 24 causes the audio record display screen P3 to display the generated audio data.

The audio record display screen P3 includes a reproduction button P3a for reproducing audio data. When the reproduction button P3a is pressed, the recording unit 24 reproduces the sound indicated by the audio data. The recording unit 24 reproduces the sound indicated by the audio data via, for example, a speaker mounted on the user terminal 2.

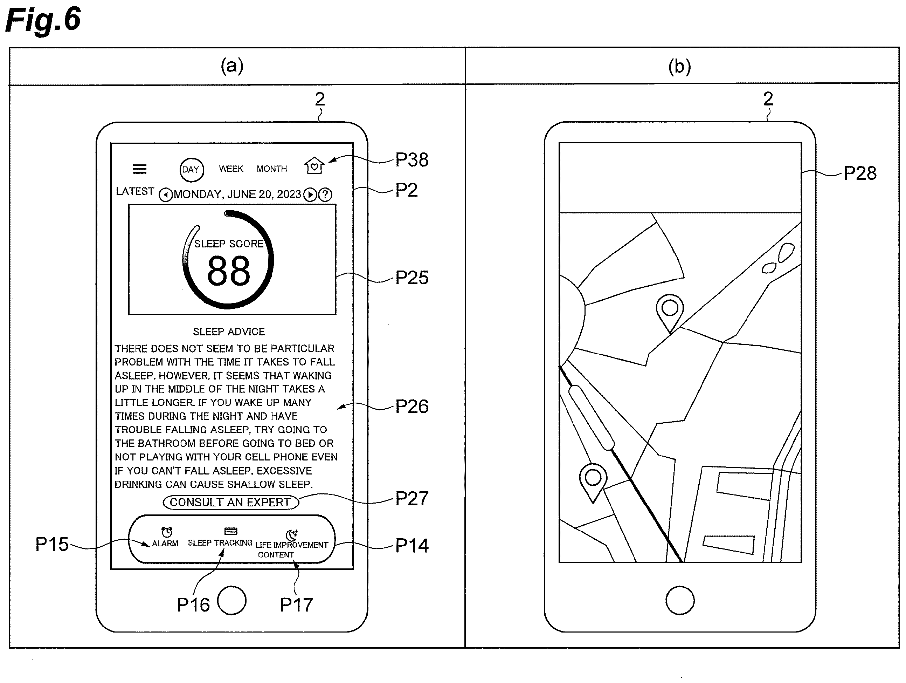

For example, when the sleep state output screen P2 shown in FIG. 4 is scrolled, the user terminal 2 displays the sleep state output screen P2 shown in (a) of FIG. 6. The sleep state output screen P2 includes a sleep score display unit P25, an advice display unit P26, and a specialist consultation unit P27. The sleep score display unit P25 is a part that displays the sleep score calculated by the sleep state output unit 23. The sleep score display unit P25 includes, for example, an annular graph showing the result of the sleep score. The annular graph expresses the result of the sleep score by the length of the annulus, for example. The annular graph is displayed color-coded with a different color for every 20% of the length of one circumference, for example. The annular graph is displayed color-coded by gradation, for example. The sleep state output unit 23 causes the sleep score display unit P25 to display the calculated sleep score.

The advice display unit P26 is a part that displays advice according to the sleep of the user state. The advice display unit P26 displays, for example, the quality of the sleep of the user onset. The advice display unit P26 displays, for example, the amount of the number of mid-sleep awakenings. The advice display unit P26 displays, for example, behavior during awakening effective for reducing the number of mid-sleep awakenings. In this way, the content providing unit 26 causes the advice display unit P26 to display advice according to the sleep of the user state.

The specialist consultation unit P27 is a part for displaying the position of a sleep consultation office. When the specialist consultation unit P27 is pressed, as shown in (b) of FIG. 6, the user terminal 2 displays a consultation office display screen P28 as a map. The consultation office display screen P28 is a screen showing the position of a sleep consultation office. The consultation office display screen P28 displays, for example, the position of a sleep consultation office located within a predetermined distance from the current location of the user as a map. The content providing unit 26 may acquire the current location of the user by, for example, a GPS (Global Positioning System) function. The content providing unit 26 causes the consultation office display screen P28 to be displayed from, for example, information indicating the position of a sleep consultation office set in advance.

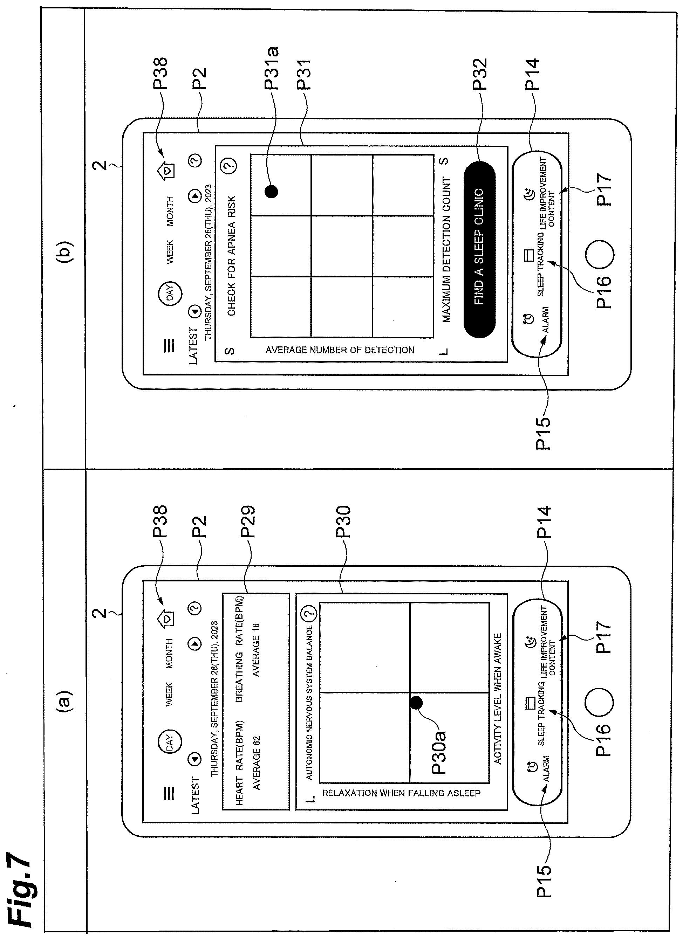

For example, when the sleep state output screen P2 shown in FIG. 4 is scrolled, the user terminal 2 displays the sleep state output screen P2 shown in FIG. 7. As shown in (a) of FIG. 7, the sleep state output screen P2 includes a heart rate/respiration display unit P29 and an autonomic nerve display unit P30. The heart rate/respiration display unit P29 is a part that displays an average value of the heart rate and an average value of the respiratory rate during the sleep of the user. The sleep state acquisition unit 22 acquires a value obtained by dividing the heart rate during sleep by the acquired sleep time from the heart rate information acquired by the analysis server 13 as the average value of the heart rate during the sleep of the user. The sleep state acquisition unit 22 causes the heart rate/respiration display unit P29 to display the acquired average value of the heart rate. The sleep state acquisition unit 22 acquires a value obtained by dividing the respiratory rate during sleep by the acquired sleep time from the respiration information acquired by the analysis server 13 as the average value of the respiratory rate during the sleep of the user. The sleep state acquisition unit 22 causes the heart rate/respiration display unit P29 to display the acquired average value of the respiratory rate.

The autonomic nerve display unit P30 is a part that displays the state of the autonomic nerve of the user. For example, the autonomic nerve display unit P30 is displayed as a two-axis graph. The vertical axis of the autonomic nerve display unit P30 indicates the relaxation level during falling asleep. The horizontal axis of the autonomic nerve display unit P30 indicates the active level during awakening. The sleep state output unit 23 causes a point P30a to be displayed on the autonomic nerve display unit P30 from the relaxation level during falling asleep and the active level during awakening acquired by the sleep state acquisition unit 22. The sleep state output unit 23 causes the autonomic nerve display unit P30 to be displayed from the state of the autonomic nerve acquired by the sleep state acquisition unit 22.

When causing the autonomic nerve display unit P30 to display the states of the autonomic nerve of a plurality of days, the sleep state output unit 23 may cause the autonomic nerve display unit P30 shown in (a) of FIG. 7 to display a plurality of points P30a, for example. One point P30a indicates, for example, the state of the autonomic nerve on a certain day.

As shown in (b) of FIG. 7, the sleep state output screen P2 includes a health risk display unit P31 and a sleep outpatient search unit P32. The health risk display unit P31 is a part that displays the health risk due to the abnormal respiratory state of the user. For example, the health risk display unit P31 is displayed as a two-axis graph. The vertical axis of the health risk display unit P31 indicates the average number of detections. The horizontal axis of the health risk display unit P31 indicates the maximum number of detections. The sleep state output unit 23 causes a point P31a to be displayed on the health risk display unit P31 from the average number of detections and the maximum number of detections acquired by the sleep state acquisition unit 22.

When causing the health risk display unit P31 to display health risks due to abnormal respiratory states of a plurality of days, the sleep state output unit 23 may cause the health risk display unit P31 shown in (b) of FIG. 7 to display a plurality of points P31a, for example. One point P31a indicates, for example, a health risk on a certain day.

The sleep outpatient search unit P32 is a part for displaying the position of a hospital having knowledge regarding sleep. When the sleep outpatient search unit P32 is pressed, the user terminal 2 displays a screen showing the position of a hospital. The screen showing the position of a hospital displays, for example, the position of a hospital located within a predetermined distance from the current location of the user. The content providing unit 26 acquires the position of a hospital included in the sleep consultation office from, for example, information indicating the position of a sleep consultation office set in advance. The content providing unit 26 causes the screen to be displayed from the acquired position of the hospital.

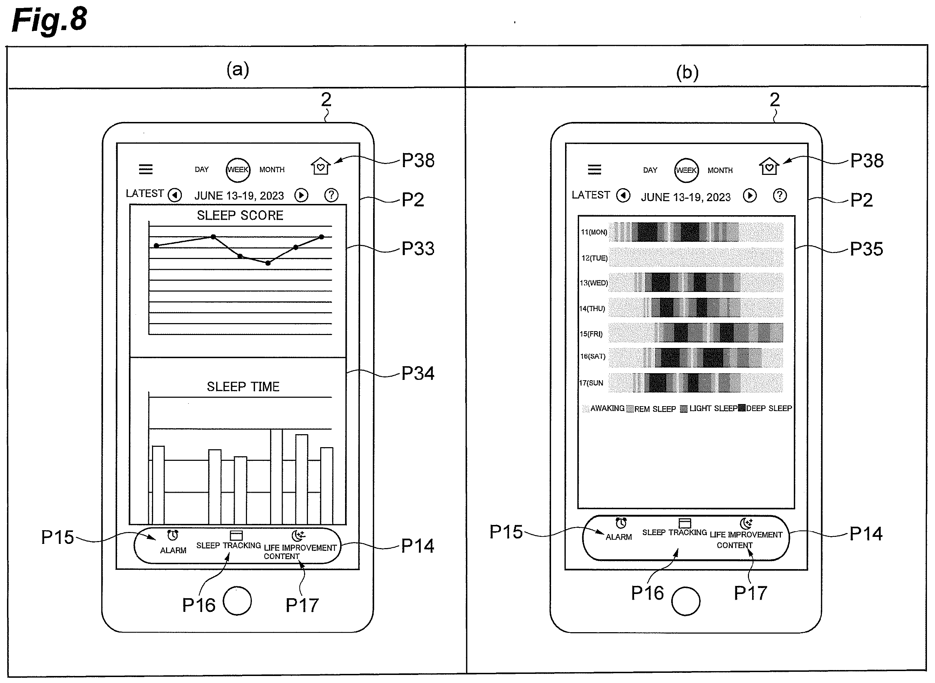

The sleep state output screen P2 displaying the sleep state for each predetermined period will be described. For example, when a button "Week" displayed at the top of the sleep state output screen P2 shown in FIG. 4 is pressed, the user terminal 2 displays the sleep state output screen P2 shown in (a) of FIG. 8. The sleep state output screen P2 includes a sleep score display unit P33 and a sleep time display unit P34. The sleep score display unit P33 is a part that displays sleep scores for a predetermined period. The "predetermined period" is one week as an example. For example, the sleep score display unit P33 is displayed as a two-axis graph. The vertical axis of the sleep score display unit P33 indicates the sleep score. The horizontal axis of the sleep score display unit P33 indicates the date. The sleep score display unit P33 displays, for example, sleep scores for one week by a line graph. The sleep state output unit 23 causes the sleep score display unit P33 to be displayed from the calculated sleep scores.

The sleep time display unit P34 is a part that displays sleep times for a predetermined period. For example, the sleep time display unit P34 is displayed as a two-axis graph. The vertical axis of the sleep time display unit P34 indicates the sleep time. The horizontal axis of the sleep time display unit P34 indicates the date. The sleep time display unit P34 displays, for example, sleep times for one week by a bar graph. The sleep state output unit 23 causes the sleep time display unit P34 to be displayed from the sleep times acquired by the sleep state acquisition unit 22.

For example, when the sleep state output screen P2 shown in (a) of FIG. 8 is scrolled, the user terminal 2 displays the sleep state output screen P2 shown in (b) of FIG. 8. The sleep state output screen P2 includes a sleep stage display unit P35. The sleep stage display unit P35 is a part that displays a time change of the sleep stage during the sleep of the user. The sleep stage display unit P35 displays, for example, the time change of the sleep stage for each day. The sleep stage display unit P35 displays, for example, the sleep stage during the sleep of the user color-coded. The sleep stage display unit P35 displays the sleep of the user stage in four stages of an awakening stage, a REM sleep stage, a light sleep stage, and a deep sleep stage, for example. The sleep state output unit 23 causes the sleep stage display unit P35 to be displayed from the sleep stage acquired by the sleep state acquisition unit 22.

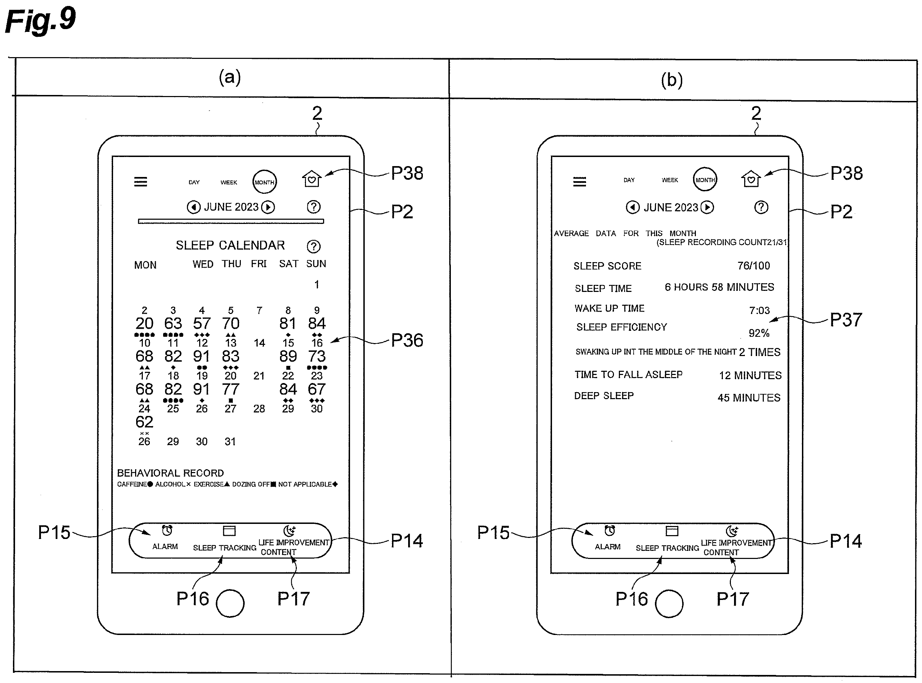

For example, when a button "Month" displayed on the sleep state output screen P2 shown in (a) of FIG. 8 is pressed, the user terminal 2 displays the sleep state output screen P2 shown in (a) of FIG. 9. The sleep state output screen P2 includes a sleep calendar display unit P36. The sleep calendar display unit P36 is a part that displays sleep scores and action records for a predetermined period. The "predetermined period" is one month as an example. The sleep calendar display unit P36 displays sleep scores for the predetermined period calculated by the sleep state output unit 23 and action records for the predetermined period acquired by the sleep state acquisition unit 22. The sleep calendar display unit P36 displays the action records divided by symbols. The sleep state output unit 23 causes the sleep calendar display unit P36 to display the calculated sleep scores. The sleep state acquisition unit 22 causes the sleep calendar display unit P36 to display the acquired action records divided by symbols.

The sleep state output screen P2 shown in (a) of FIG. 9 may include, for example, a part displaying sleep scores for one month. The part may display sleep scores for one month by a line graph, for example. The sleep state output unit 23 may cause the part to be displayed from the calculated sleep scores.

For example, when the sleep state output screen P2 shown in (a) of FIG. 9 is scrolled, the user terminal 2 displays the sleep state output screen P2 shown in (b) of FIG. 9. The sleep state output screen P2 includes an average data display unit P37. The average data display unit P37 displays, for example, at least one of an average value of sleep scores, an average value of sleep times, an average value of wake-up times, an average value of sleep efficiency, an average value of the number of mid-sleep awakenings, an average value of sleep onset latencies, and an average value of deep sleep times in a predetermined period. The sleep state output unit 23 causes the average data display unit P37 to display the calculated average value of sleep scores in the predetermined period. The sleep state output unit 23 causes the average data display unit P37 to display the average values of sleep times, wake-up times, sleep efficiency, number of mid-sleep awakenings, and sleep onset latencies acquired by the sleep state acquisition unit 22. The sleep state output unit 23 acquires a time during which the sleep stage was a sleep stage deeper than a predetermined stage during sleep from the sleep stage acquired by the sleep state acquisition unit 22. The sleep state output unit 23 causes the average data display unit P37 to display an average value in the predetermined period of the time during which acquired the sleep stage was a sleep stage deeper than the predetermined stage as the average value of deep sleep times.

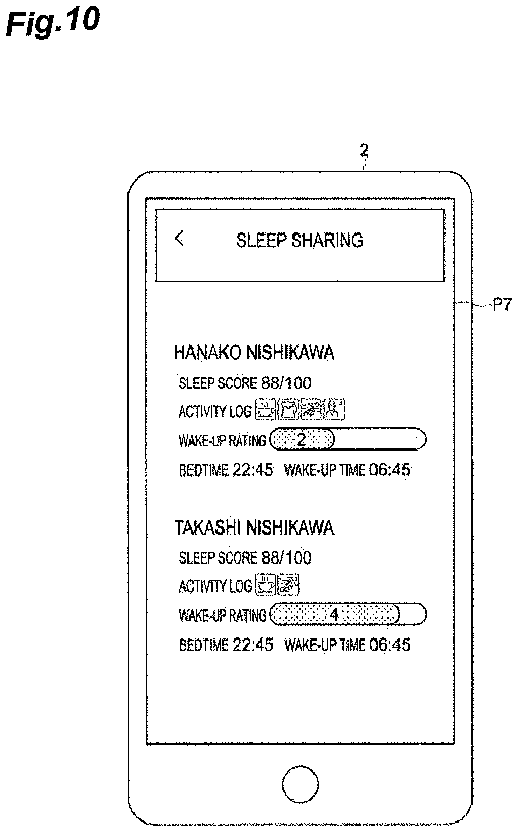

The sleep state output screen P2 includes a sleep sharing screen instruction unit P38. For example, when the sleep sharing screen instruction unit P38 is pressed, the user terminal 2 displays a sleep sharing result screen P7. FIG. 10 is a diagram showing an example of the sleep sharing result screen P7. The sleep sharing result screen P7 is a screen showing a result of outputting the sleep of the user state toward another user. The sleep sharing result screen P7 displays the sleep score, action record, post-awakening evaluation, bedtime, and wake-up time for each user belonging to the group.

The sleep state acquisition unit 22 causes the sleep sharing result screen P7 to display the acquired action record and post-awakening evaluation. The sleep state acquisition unit 22 causes the sleep sharing result screen P7 to display the acquired bedtime and wake-up time. The sleep state output unit 23 causes the sleep sharing result screen P7 to display the calculated sleep score. The sharing unit 29 outputs the sleep of the user state to another user belonging to the group including the user. The sharing unit 29 causes the sleep of the user state to be displayed on the sleep sharing result screen P7 in the user terminal 2 of another user as an output.

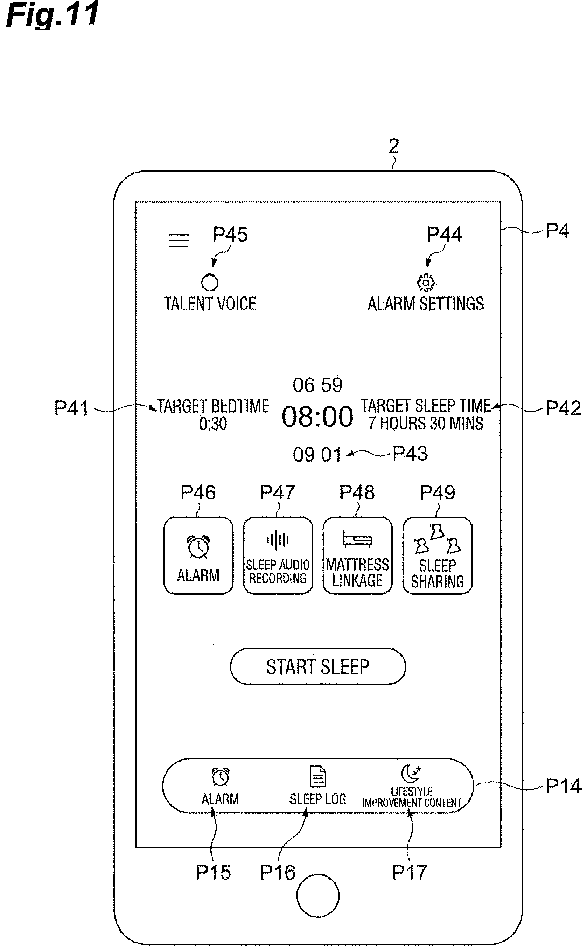

When the alarm selection unit P15 in the menu bar P14 (see FIG. 4 etc.) is pressed, the user terminal 2 displays an alarm setting screen P4. FIG. 11 is a diagram showing an example of the alarm setting screen P4. For example, even if the alarm selection unit P15 is pressed, the menu bar P14 remains without disappearing on the alarm setting screen P4. The alarm setting screen P4 is a screen for the user to select the type of audio content provided to the user at the time of falling asleep and the time of awakening of the user. The alarm setting screen P4 includes, for example, a target bedtime display unit P41 that displays a target bedtime, a target sleep time display unit P42 that displays a target sleep time, and a reproduction time display unit P43 that displays a reproduction time at which audio content is reproduced. The target bedtime, the target sleep time, and the reproduction time are set by the user, for example.

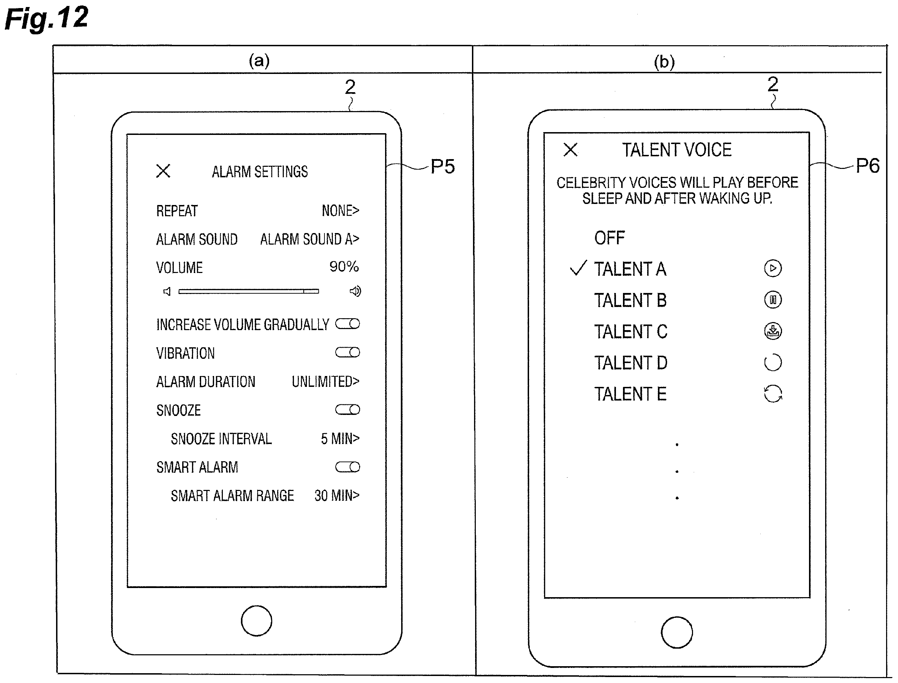

The alarm setting screen P4 includes an alarm setting unit P44 and a talent voice setting unit P45. When the alarm setting unit P44 is pressed, the user terminal 2 displays an alarm content setting screen P5. (a) of FIG. 12 is a diagram showing an example of the alarm content setting screen P5. On the alarm content setting screen P5, the user selects the type of audio content. On the alarm content setting screen P5, for example, the user selects, as the type of audio content, presence or absence of repetition of audio content, type of sound (alarm sound) in audio content, volume of audio content, whether to gradually increase the volume of audio content, presence or absence of vibration, and reproduction time of audio content. On the alarm content setting screen P5, the user selects, for example, presence or absence of snooze, snooze interval, presence or absence of smart alarm, and range of smart alarm as the type of audio content.

The smart alarm is a function of controlling the timing of reproducing audio content according to the sleep of the user stage. The range of the smart alarm means the time width of a reproduction time zone. The reproduction time zone is a time zone including the time at which audio content is reproduced by the content providing unit 26. In other words, when the smart alarm is set to present, the content providing unit 26 reproduces audio content at any time included in the reproduction time zone.

For example, when the user sets the smart alarm to present, the content providing unit 26 determines the timing to reproduce audio content from the sleep stage acquired by the sleep state acquisition unit 22 and the selected range of the smart alarm. More specifically, the content providing unit 26 acquires, for example, a time zone past by the time width of the selected range of the smart alarm from the preset reproduction time (see the reproduction time display unit P43 in FIG. 11) as the reproduction time zone. The content providing unit 26 determines a timing at which the sleep stage first becomes a sleep stage shallower than a predetermined stage in the acquired reproduction time zone as the timing to reproduce audio content. The content providing unit 26 reproduces audio content at the determined timing. In this case, audio content can be reproduced to the user at a timing when the sleep of the user is shallower than the predetermined stage. As a result, the user can awaken more smoothly.

For example, when there is no timing at which the sleep stage becomes a sleep stage shallower than the predetermined stage in the acquired reproduction time zone (when remaining in deep sleep), the content providing unit 26 may determine the set reproduction time as the timing to reproduce audio content. For example, when the user sets the smart alarm to absent, the content providing unit 26 may reproduce audio content at the selected reproduction time.

(b) of FIG. 12 is a diagram showing an example of a talent voice setting screen P6. As shown in FIG. 11 and (b) of FIG. 12, when the talent voice setting unit P45 is pressed, the user terminal 2 displays the talent voice setting screen P6. The talent voice setting screen P6 is a screen for the user to select a person (talent) who transmits audio content provided to the user. On the talent voice setting screen P6, the user selects, for example, a person who utters a voice as the type of audio content. In the present embodiment, the person is, for example, a celebrity such as an entertainer or an athlete.

When the user selects audio content (e.g., alarm sound) on the alarm content setting screen P5, the reception unit 25 receives the audio content selected on the alarm content setting screen P5. When the user selects audio content (e.g., which talent to use) on the talent voice setting screen P6, the reception unit 25 receives the audio content selected on the talent voice setting screen P6.

The alarm setting screen P4 includes an alarm instruction unit P46, an audio record instruction unit P47, a mattress cooperation instruction unit P48, and a sleep sharing instruction unit P49. The alarm instruction unit P46 is a part for instructing whether or not to reproduce audio content at the time of awakening of the user. For example, by pressing the alarm instruction unit P46, the reproduction function of audio content is enabled or disabled. When the reproduction function of audio content is enabled, the content providing unit 26 reproduces audio content of the type selected on at least one of the alarm content setting screen P5 and the talent voice setting screen P6 at the time of awakening of the user.

The audio record instruction unit P47 is a part for the user to instruct whether or not to record sounds emitted in the user and around the user during sleep of the user. For example, by pressing the audio record instruction unit P47, the sound recording function is enabled or disabled. When the sound recording function is enabled, the recording unit 24 starts recording sounds emitted in the user and around the user. When the audio record instruction unit P47 is pressed, the recording unit 24 may start recording the sound at the sleep of the user onset time acquired by the sleep state acquisition unit 22.

The mattress cooperation instruction unit P48 is a part for the user to instruct whether or not to cooperate the sleep analysis application 20 and the sensor unit 11 attached to the mattress 10. By pressing the mattress cooperation instruction unit P48, the cooperation function between the sleep analysis application 20 and the sensor unit 11 is enabled or disabled. When the cooperation function is enabled, the sleep analysis application 20 starts cooperation with the sensor unit 11. Cooperation between the user terminal 2 and the sensor unit 11 may be realized by a known communication means such as Bluetooth (registered trademark).

The sleep sharing instruction unit P49 is a part for the user to instruct whether or not to output the sleep of the user state toward another user. By pressing the sleep sharing instruction unit P49, the sharing function of the sleep state is enabled or disabled. When the sharing function of the sleep state is enabled, the sharing unit 29 outputs the sleep of the user state to another user belonging to the group including the user.

The content of the aforementioned alarm setting screen P4 is not particularly limited and can be changed as appropriate. For example, the alarm setting screen P4 may not include at least one of the alarm instruction unit P46, the audio record instruction unit P47, the mattress cooperation instruction unit P48, and the sleep sharing instruction unit P49.

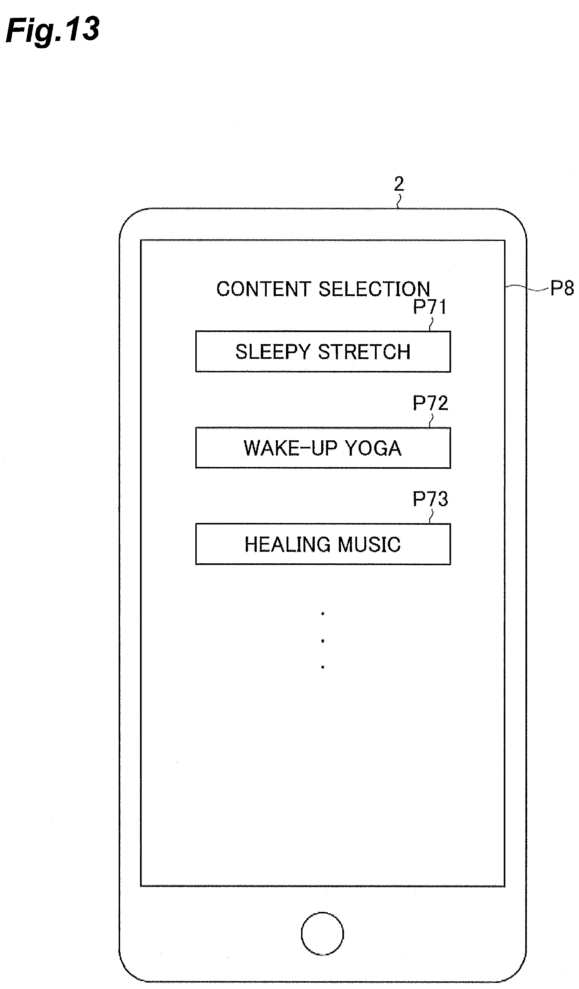

When the content selection unit P17 in the menu bar P14 (see FIG. 11 etc.) is pressed, the user terminal 2 displays a content selection screen P8. FIG. 13 is a diagram showing an example of the content selection screen P8. The content selection screen P8 is a screen for the user to select life improvement content. The content selection screen P8 includes a first content selection unit P71, a second content selection unit P72, and a third content selection unit P73.

For example, the first content selection unit P71 is a part for the user to select a video of stretching as life improvement content provided by the content providing unit 26. The stretching is, for example, considered to make it easier to fall asleep smoothly by performing it before falling asleep. When the first content selection unit P71 is pressed, the content providing unit 26 causes a video content screen P9 to be displayed.

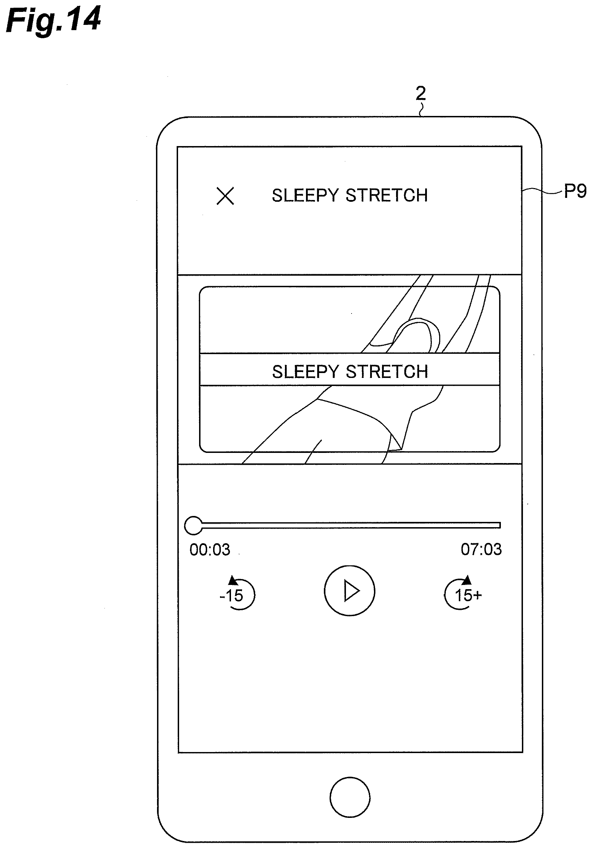

FIG. 14 is a diagram showing an example of the video content screen P9. The video content screen P9 displays, for example, a video of stretching. The user can perform stretching before falling asleep while watching the video content screen P9, for example.

As shown in FIG. 13, for example, the second content selection unit P72 is a part for the user to select a video of yoga as life improvement content provided by the content providing unit 26. The yoga is, for example, considered to make it easier to awaken smoothly by performing it after awakening. When the second content selection unit P72 is pressed, the content providing unit 26 causes a video of yoga to be displayed.

For example, the third content selection unit P73 is a part for the user to select music as life improvement content provided by the content providing unit 26. The music is considered to have a relaxing effect, such as healing music. When the third content selection unit P73 is pressed, the content providing unit 26 reproduces music.

As shown in FIG. 1, the service output system 1 comprises a store terminal 41, an individual customer information server 42, a customer information server 43, a point management server 44, and a store information server 45.

In the present embodiment, the service output system 1 comprises a plurality of store terminals 41. The store terminal 41 is a terminal used by a store. In the present embodiment, the store terminal 41 may be a terminal of the same type as the user terminal 2, or may be a terminal of a different type from the user terminal 2. The store terminal 41 is operated by, for example, a user who visited the store. To the store terminal 41, for example, identification information assigned by the identification information assigning unit 51 is input by the user.

The store is a store that presents information on a product to a user and prompts purchase of the product. Stores include specialty stores selling specific products, affiliated stores (franchises) belonging to a company, tenant stores in commercial facilities such as department stores or shopping centers, and mass retailers that purchase products and sell products.

The store terminal 41 is capable of communicating with a main body unit 100 (see FIG. 2) that conducts transactions with a plurality of stores. The main body unit 100 is a company (including an organization, a company, a corporation, or an individual) that sells products to users via stores. The main body unit 100 includes a main body organization that manufactures and manages inventory of products handled by stores, a franchiser that supervises affiliated stores, and a headquarters organization that supervises mass retailers.

When the user purchases a product by the store terminal 41, the store terminal 41 transmits order information of the user to the main body unit 100 together with the identification information of the user. The order information is information specifying the product ordered by the user. The order information includes the amount of money of the product ordered by the user. When receiving the order information from the store terminal 41, the main body unit 100 provides the ordered product to the user identified by the identification information included in the received order information.

The main body unit 100 specifies the product ordered by the user from, for example, the order information received from the store terminal 41. The main body unit 100 identifies and specifies the user who ordered the product from, for example, the identification information received from the store terminal 41. The main body unit 100 acquires the name and address of the user from basic information of the specified user, for example. The main body unit 100 provides the product to the user by sending the ordered product to the acquired address of the user. The main body unit 100 may send the product to the user by means such as mail, for example.

The store terminal 41 is capable of communicating with the individual customer information server 42. When transmitting the order information to the main body unit 100, the store terminal 41 transmits the order information to the individual customer information server 42 (described later) arranged in the store where the store terminal 41 is arranged.

In the present embodiment, a money calculator is arranged in the store in addition to the store terminal 41. The money calculator is used, for example, when the user pays the consideration for the product after the store terminal 41 receives the order of the user. The money calculator is, for example, a money calculator using a POS (Point Of Sales) system. In this case, the money calculator may be capable of cooperating with the store terminal 41 by the POS system. However, the function of the money calculator may be included in the function of the store terminal 41. In this case, the money calculator may not be provided in the store.

In the present embodiment, the service output system 1 comprises the same number of individual customer information servers 42 as the number of stores. The individual customer information server 42 is arranged for each store, for example. The number of individual customer information servers 42 is not particularly limited. The individual customer information server 42 is capable of communicating with the customer information server 43 and the point management server 44. In the present embodiment, each of the plurality of individual customer information servers 42 is capable of communicating with the customer information server 43 and the point management server 44.

The individual customer information server 42 transmits the order information received from the store terminal 41 to the customer information server 43. The individual customer information server 42 includes store identification information uniquely specifying the store in the order information and transmits it to the customer information server 43. The store identification information is set in advance, for example.

Based on the order information received from the store terminal 41, the individual customer information server 42 generates point information indicating points according to the amount of money of the product included in the order information. The points according to the amount of money may be, for example, a value obtained by multiplying the amount of money by a predetermined coefficient. The individual customer information server 42 transmits the aforementioned identification information to the point management server 44 together with the generated point information.

In the present embodiment, the user can purchase a product not only by the store terminal 41 but also by the user terminal 2. The user terminal 2 is capable of accessing a shopping site where products can be purchased, for example. When the user purchases a product by the user terminal 2, the user terminal 2 transmits order information of the user to the main body unit 100 together with the identification information of the user. When transmitting the order information to the main body unit 100, the user terminal 2 transmits the order information to the customer information server 43.

In the present embodiment, the user terminal 2 generates point information indicating points according to the amount of money of the product included in the order information. The user terminal 2 transmits the generated point information to the point management server 44 together with the identification information.

The customer information server 43 is capable of communicating with the user terminal 2. The customer information server 43 generates purchase information indicating the product purchased by the user from the order information received from the individual customer information server 42 or the user terminal 2. The purchase information is, for example, a history of products purchased by the user. The customer information server 43 stores the generated purchase information. The customer information server 43 stores the purchase information for each store from the store identification information transmitted from the individual customer information server 42. The customer information server 43 transmits the generated purchase information to the user terminal 2. The customer information server 43 stores the basic information received from the user terminal 2. The customer information server 43 receives a password from the user terminal 2 and stores the password received from the user terminal 2. When receiving a changed password from the user terminal 2, the customer information server 43 updates the stored password to the changed password.

The point management server 44 manages points of the user. The points may be points that can be exchanged for cash, or points that cannot be exchanged for cash and can be used when ordering products from the next time onwards. The points usable when ordering are, for example, an upper limit of an amount that can be discounted from a predetermined amount when purchasing a product of the predetermined amount. The point management server 44 manages points for each user.

The point management server 44 is capable of communicating with the user terminal 2. When receiving point information from the individual customer information server 42 or the user terminal 2, the point management server 44 assigns points according to the received point information to the user. The point management server 44 assigns points indicated by the received point information to the user. The point management server 44 stores held points which are points held by the user. The point management server 44 transmits the held points to the user terminal 2 and the store terminal 41.

The store information server 45 stores store position information indicating the position of a store. The store position information is set in advance, for example. The store information server 45 is capable of communicating with the customer information server 43, for example. The store information server 45 transmits the store position information to the customer information server 43. The store information server 45 transmits the store position information to the user terminal 2 via the customer information server 43.

In the present embodiment, the service output system 1 further comprises an analysis device 3. The analysis device 3 is a device that specifies a service recommended to the user and outputs the specified service toward the user. The analysis device 3 is capable of communicating with the user terminal 2. Details of the analysis device 3 will be described later.

The service proposal application 30 will be described. As shown in FIG. 2, the service proposal application 30 has, as its functional configuration, an identification information assigning unit 51, a purchase information acquisition unit 52, and a point assigning unit 53.

The identification information assigning unit 51 assigns identification information for each user. For example, when the user starts the service proposal application 30 for the first time on the user terminal 2, the identification information assigning unit 51 receives an input of basic information by the user. The basic information includes, for example, the name, address, telephone number, and family structure of the user. The identification information assigning unit 51 generates identification information. The identification information assigning unit 51 sets and assigns the generated identification information to the user. The identification information assigning unit 51 generates, for example, a password of the user. The identification information assigning unit 51 generates, for example, a character string consisting of a predetermined number of alphabets and a predetermined number of numerals as the password.

The identification information assigning unit 51 links each of the generated password and the received basic information to the identification information assigned to the user. The identification information assigning unit 51 transmits the identification information, the password linked to the identification information, and the basic information linked to the identification information to the customer information server 43.

The identification information assigning unit 51 may prompt the user to change the password, for example. In this case, the identification information assigning unit 51 causes the display of the user terminal 2 to display a message with content requesting a change of the password, for example. The identification information assigning unit 51 receives an input of a changed password by the user. The identification information assigning unit 51 transmits the received changed password to the customer information server 43.

The purchase information acquisition unit 52 acquires purchase information. The purchase information acquisition unit 52 receives and acquires purchase information from the customer information server 43. The purchase information acquisition unit 52 transmits the acquired purchase information to the analysis device 3.

The point assigning unit 53 acquires the sleep state output from the sleep state output unit 23. The point assigning unit 53 assigns points according to the sleep state output from the sleep state output unit 23 to the user. The point assigning unit 53 generates point information indicating points according to the acquired sleep state. The point assigning unit 53 may generate point information indicating points of a value equal to the sleep score calculated by the sleep state output unit 23, for example. The point assigning unit 53 transmits the generated point information to the point management server 44 together with the identification information.

The service proposal application 30 causes the user terminal 2 to display the identification information assigned by the identification information assigning unit 51. The service proposal application 30 causes the user terminal 2 to display the held points received from the point management server 44. The service proposal application 30 transmits the sleep state shared by the sleep state output unit 23 to the analysis device 3.

The service proposal application 30 causes the user terminal 2 to display topics related to sleep, for example. The topics include, for example, information indicating products for improving the quality of sleep. The topics include, for example, information indicating habits for improving the quality of sleep. The service proposal application 30 may display topics preset in the service proposal application 30 on the user terminal 2. The service proposal application 30 may receive topics from a website, for example. The service proposal application 30 may cause the user terminal 2 to display the topics received from the website, for example.

The service proposal application 30 may cause the user terminal 2 to display the position of a store, for example. The service proposal application 30 receives store position information from the store information server 45 via the customer information server 43, for example. The service proposal application 30 may cause the user terminal 2 to display the position of a store from the received store position information, for example.

The service proposal application 30 may cause the user terminal 2 to display information indicating a service provided by the main body unit 100, for example. The service provided by the main body unit 100 is, for example, a rental service of products. The information indicating a service is, for example, an advertisement proposing a service to the user. The service proposal application 30 may acquire information indicating a service from the main body unit 100, for example. The service proposal application 30 may cause the user terminal 2 to display the acquired information indicating a service.

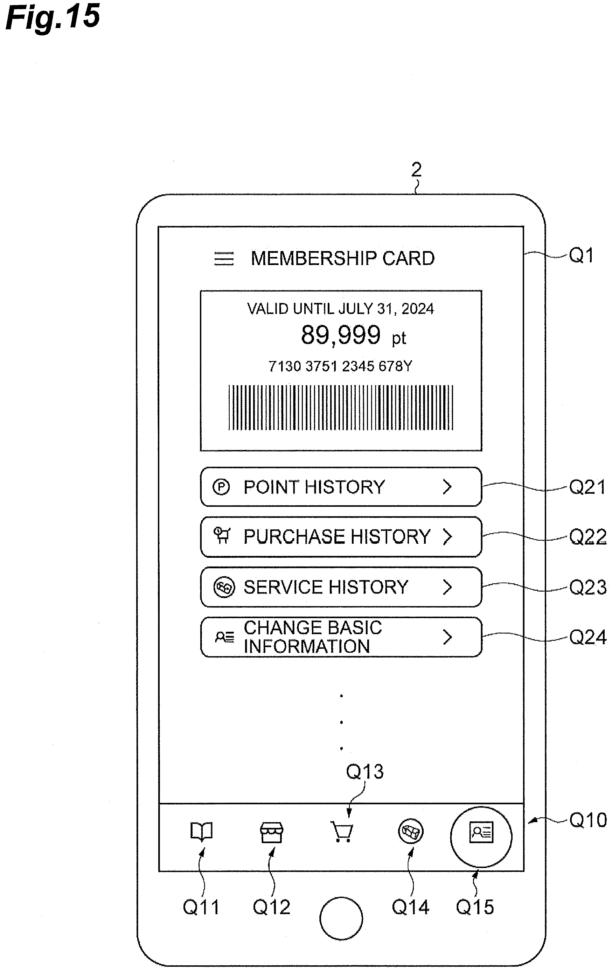

For example, when the service proposal application 30 is executed, the service proposal application 30 causes the user terminal 2 to display a member information display screen Q1. FIG. 15 is a diagram showing an example of the member information display screen Q1. The member information display screen Q1 includes a menu bar Q10. The menu bar Q10 includes a topics instruction unit Q11, a store instruction unit Q12, a shopping instruction unit Q13, a service instruction unit Q14, and a member information instruction unit Q15.

The topics instruction unit Q11 is a part for the user to instruct display of topics related to sleep. When the topics instruction unit Q11 is pressed, the service proposal application 30 causes topics related to sleep to be displayed. The store instruction unit Q12 is a part for the user to instruct display of the position of the aforementioned store. When the store instruction unit Q12 is pressed, the service proposal application 30 causes the user terminal 2 to display the position of a store according to the store position information received from the store information server 45.

The shopping instruction unit Q13 is a part for the user to instruct access to a shopping site where products can be purchased. When the shopping instruction unit Q13 is pressed, the service proposal application 30 causes the user terminal 2 to access the shopping site. The service instruction unit Q14 is a part for the user to instruct display of a service that can be provided to the user by the main body unit 100. When the service instruction unit Q14 is pressed, the service proposal application 30 causes the user terminal 2 to access a website indicating a service provided by the main body unit 100. For example, the user can make an application for enjoying the service via the website. When the application is made by the user, for example, the service proposal application 30 transmits application information to the customer information server 43. The application information includes, for example, a history of services enjoyed by the user.

For example, the service instruction unit Q14 is displayed before the member information display screen Q1 is displayed. In this state, when the member information instruction unit Q15 is pressed, the service proposal application 30 causes the member information display screen Q1 to be displayed. The member information display screen Q1 displays, for example, the held points received from the point management server 44 and the identification information assigned by the identification information assigning unit 51. The member information display screen Q1 displays, for example, a barcode indicating the identification information. The member information display screen Q1 may display a two-dimensional code such as a QR code (registered trademark) instead of the barcode.

The member information display screen Q1 includes a point history instruction unit Q21, a purchase history instruction unit Q22, a service history instruction unit Q23, and a basic information change instruction unit Q24. The point history instruction unit Q21 is a part for instructing display of a history of points of the user. For example, when the point history instruction unit Q21 is pressed, the service proposal application 30 acquires point information from the point management server 44. The service proposal application 30 generates information indicating a history of points of the user from the acquired point information. The service proposal application 30 causes the user terminal 2 to display the generated information indicating a history of points.

The purchase history instruction unit Q22 is a part for the user to instruct display of a purchase history of the user. For example, when the purchase history instruction unit Q22 is pressed, the service proposal application 30 acquires purchase information from the customer information server 43. The service proposal application 30 causes the user terminal 2 to display the acquired purchase information.

The service history instruction unit Q23 is a part for the user to instruct display of a history of services enjoyed by the user. For example, when the service history instruction unit Q23 is pressed, the service proposal application 30 acquires application information from the customer information server 43. The service proposal application 30 causes the user terminal 2 to display the acquired application information.

The basic information change instruction unit Q24 is a part for the user to instruct display of a basic information change screen (not shown). The basic information change screen is a screen for changing the basic information of the user. When the basic information change instruction unit Q24 is pressed, the service proposal application 30 causes the user terminal 2 to display the basic information change screen. The service proposal application 30 receives changed basic information via the basic information change screen. The service proposal application 30 transmits the received changed basic information to the customer information server 43. The customer information server 43 updates the stored basic information to the changed basic information received from the service proposal application 30.

The analysis device 3 shown in FIG. 1 is, for example, a server. The analysis device 3 comprises, as an example, a processor (e.g., CPU) that executes an operating system and software (application), a main storage unit composed of ROM and RAM, an auxiliary storage unit composed of flash memory, a communication control unit composed of a wireless communication module, an input device, and an output device such as a display. However, the configuration of the analysis device 3 is not limited to the above and can be changed as appropriate. The analysis device 3 has, as its functional configuration, a service specifying unit 61 and a service output unit 62.

The service specifying unit 61 receives and acquires purchase information from the purchase information acquisition unit 52. The service specifying unit 61 receives and acquires a sleep state from the service proposal application 30. The service specifying unit 61 specifies a service recommended to the user from the acquired purchase information and sleep state. The service specifying unit 61 acquires the preference of the user from the purchase information, for example. The service specifying unit 61 specifies provision of an advertisement of a product along the acquired preference as a service. The service specifying unit 61 acquires, for example, the sleep of the user score as the sleep state. When the acquired sleep score is equal to or less than a predetermined value, the service specifying unit 61 specifies provision of an advertisement of a product capable of making sleep good as a service.

The service output unit 62 outputs the service specified by the service specifying unit 61 toward the user. In the present embodiment, the service output unit 62 transmits an e-mail indicating the service specified by the service specifying unit 61 to the user terminal 2. The service output unit 62 may display the service specified by the service specifying unit 61 on the user terminal 2 as an application screen of the service proposal application 30. The output mode of the service output unit 62 is not particularly limited.

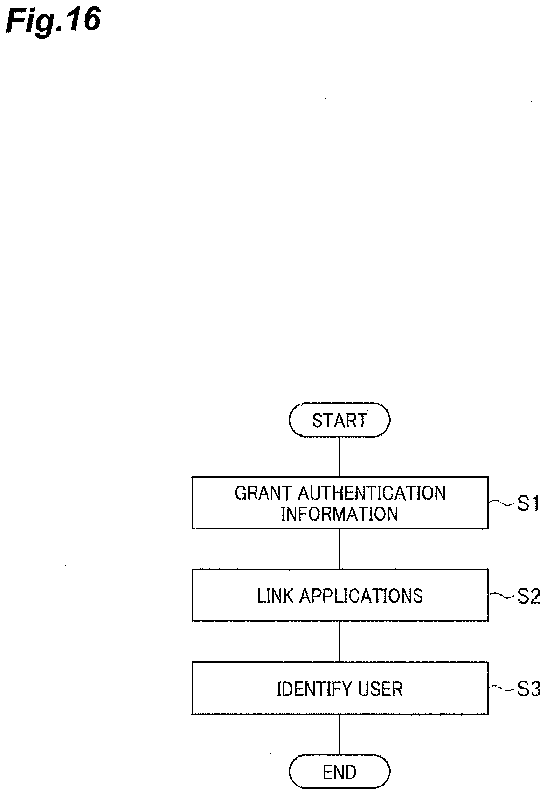

An example of the operation of the service output system 1 will be described. FIG. 16 is a flowchart showing an example of the operation of the service output system 1 for specifying a user identified by identification information. The service output system 1 executes the operation shown in FIG. 16 before acquiring the sleep of the user state. For example, when the user starts the service proposal application 30 for the first time on the user terminal 2, the identification information assigning unit 51 assigns identification information for each user (step S1). More specifically, the identification information assigning unit 51 first receives an input of basic information by the user. Then, the identification information assigning unit 51 generates identification information, and sets and assigns the generated identification information to the user.

In step S1, the identification information assigning unit 51 generates a password. Thereafter, the identification information assigning unit 51 links each of the generated password and the received basic information to the identification information assigned to the user. In step S1, the identification information assigning unit 51 may prompt the user to change the password.

The service output system 1 cooperates applications (step S2). In step S2, the service output system 1 puts the sleep analysis application 20 and the service proposal application 30 into a state capable of sharing information with each other. The user terminal 2 displays the authentication screen P1 (see FIG. 3). The user terminal 2 displays the authentication screen P1 on the display of the user terminal 2 according to instructions of a program constituting the sleep analysis application 20. The user terminal 2 receives an input of identification information and a password by the user via the authentication screen P1.

The user specifying unit 21 determines whether or not the identification information input by the user matches the identification information assigned in step S1. The user specifying unit 21 determines whether or not the password input by the user matches the password set in association with the identification information in step S1.

When it is determined that the input identification information matches the assigned identification information, and it is determined that the input password matches the set password, the sleep analysis application 20 and the service proposal application 30 enter a state capable of sharing information with each other. At this time, the user specifying unit 21 specifies the user who input the identification information and the password as the user identified by the identification information (step S3). Through the above steps, the service output system 1 specifies the user identified by the identification information.

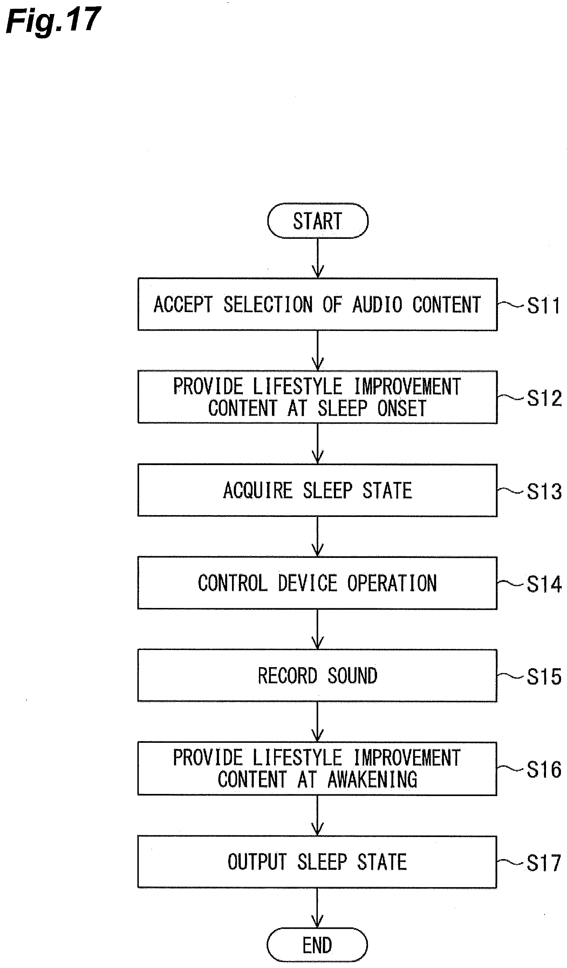

FIG. 17 is a flowchart showing an example of the operation of the service output system 1 until outputting a sleep state. Before the service output system 1 executes the operation shown in FIG. 17, first, the sensor unit 11 is attached to the mattress 10. In the present embodiment, a sensor sheet is attached to the mattress 10 as the sensor unit 11. Next, the body of the user is placed on the mattress 10.

Before the service output system 1 executes the operation shown in FIG. 17, the content server 16 stores life improvement content in advance. The life improvement content includes audio content. The content server 16 stores advice regarding the sleep of the user as life improvement content. The content server 16 stores information prompting behavior modification of the user as life improvement content.

The reception unit 25 receives a selection of audio content (step S11). In step S11, the reception unit 25 receives a selection of audio content by the user from a plurality of types of audio content set in advance. In step S11, for example, the user terminal 2 displays the alarm content setting screen P5 (see (a) of FIG. 12). The reception unit 25 receives, for example, audio content selected on the alarm content setting screen P5.

The content providing unit 26 provides life improvement content at the time of falling asleep of the user (step S12). In step S12, the content providing unit 26 provides, for example, the audio content selected in step S11. In step S11, when the user does not select life improvement content provided at the time of falling asleep, the service output system 1 may omit execution of step S12 and execute step S13.

After the user falls asleep, the analysis server 13 analyzes the sleep of the user. The analysis server 13 acquires the sleep of the user state from the acquired respiration information, body movement information, and heart rate information. The analysis server 13 acquires, as the sleep state, the sleep of the user stage, sleep time, sleep onset latency, number of mid-sleep awakenings, sleep efficiency, state of autonomic nerve, and health risk due to abnormal respiratory state. The analysis server 13 acquires the relaxation level during falling asleep and the active level during awakening as the state of the autonomic nerve. The analysis server 13 acquires the average number of detections and the maximum number of detections as the health risk due to the abnormal respiratory state. The analysis server 13 transmits the acquired sleep state to the short-term data server 14. At this time, the short-term data server 14 stores the sleep state received from the analysis server 13.

The sleep state acquisition unit 22 acquires the sleep state acquired by the analysis server 13 (step S13). In step S13, the sleep state acquisition unit 22 transmits an output command to the short-term data server 14 at a predetermined timing. When receiving the output command from the sleep state acquisition unit 22, the short-term data server 14 transmits the stored sleep state to the sleep state acquisition unit 22. The sleep state acquisition unit 22 receives and acquires the sleep state from the short-term data server 14.

The device control unit 27 controls the operation of the device 12 from the sleep state acquired in step S13 (step S14). The recording unit 24 records sounds emitted in the user and around the user during sleep of the user (step S15). In step S15, the recording unit 24 records, for example, at least one of the snoring of the user and an operation sound of a device. The recording unit 24 records the recorded sound to generate an audio record.

In step S15, the recording unit 24 generates audio data indicating sound in a time zone in which a sound having a volume equal to or greater than a predetermined amount occurred from the generated audio record. The recording unit 24 causes the audio record display screen P3 (see FIG. 5) to display the generated audio data. For example, when the reproduction button P3a on the audio record display screen P3 is pressed after the user wakes up, the recording unit 24 reproduces the sound indicated by the audio data.

The content providing unit 26 provides (e.g., reproduces) life improvement content at the time of awakening of the user (step S16). The content providing unit 26 may reproduce, for example, the audio content selected in step S11. In this case, the content providing unit 26 reproduces the audio content at the time of awakening of the user by reproducing the audio content at the reproduction time set in step S11.

In step S16, the content providing unit 26 may provide audio content according to the sleep state acquired in step S13. In this case, the content providing unit 26 adjusts the volume of the audio content according to the sleep state acquired in step S13. The content providing unit 26 acquires, for example, the sleep score calculated in step S13. In step S16, the content providing unit 26 sets the volume of the audio content to a predetermined first volume when determining that the sleep score is equal to or greater than a predetermined value, for example, and sets the volume of the audio content to a second volume larger than the first volume when determining that the sleep score is not equal to or greater than the predetermined value.

In step S16, the content providing unit 26 may provide advice according to the sleep state acquired in step S13, for example. The content providing unit 26 receives and acquires advice regarding the sleep of the user from the content server 16. The content providing unit 26 determines, for example, whether or not the sleep onset latency acquired in step S13 is equal to or longer than a predetermined time. When determining that the sleep onset latency is equal to or longer than the predetermined time, for example, the content providing unit 26 receives advice to the effect that there is a tendency to have difficulty falling asleep among advice regarding sleep from the content server 16. The content providing unit 26 provides the received advice to the effect that there is a tendency to have difficulty falling asleep.

In step S16, the content providing unit 26 may provide information prompting behavior modification according to the sleep state acquired in step S13. In this case, the content providing unit 26 acquires the sleep score calculated by the sleep state output unit 23. When determining that the acquired sleep score is equal to or less than a predetermined value, for example, the content providing unit 26 receives information prompting to consult a sleep specialist among information prompting behavior modification from the content server 16. The content providing unit 26 provides the received information prompting to consult a sleep specialist.

In step S16, the content providing unit 26 may provide life improvement content according to the audio record generated in step S15. The content providing unit 26 extracts, for example, the volume, length, and frequency of the snoring of the user from the audio record generated in step S15. The content providing unit 26 calculates the severity of snoring from the extracted volume, length, and frequency of the snoring of the user. The content providing unit 26 provides life improvement content according to the calculated severity of snoring, for example.

The content providing unit 26 determines, for example, whether or not the calculated severity of snoring is equal to or greater than a predetermined value. When determining that the calculated severity of snoring is equal to or greater than the predetermined value, for example, the content providing unit 26 provides information prompting to consult a sleep specialist as information prompting behavior modification.

In step S11, when the user does not select life improvement content provided at the time of awakening, the service output system 1 may omit execution of step S16 and execute step S17.

After the user wakes up, the sleep state acquisition unit 22 acquires a post-awakening evaluation of the user. The sleep state acquisition unit 22 receives, for example, an input of the post-awakening evaluation by the user after awakening and acquires the received post-awakening evaluation. The sleep state acquisition unit 22 receives, for example, an action record input on a predetermined screen. The sleep state acquisition unit 22 causes the action record display unit P21 (see FIG. 4) to display the received action record. The sleep state acquisition unit 22 attaches a check mark to an icon corresponding to the action taken by the user from the received action record, for example.

The sleep state output unit 23 outputs the sleep state acquired in step S13 toward the user (step S17). In step S17, the sleep state output unit 23 outputs, as the sleep state, the sleep stage, sleep time, sleep onset latency, number of mid-sleep awakenings, sleep efficiency, state of autonomic nerve, and health risk due to abnormal respiratory state. The sleep state output unit 23 calculates a sleep score from the sleep state acquired in step S13. The sleep state output unit 23 outputs the calculated sleep score. The sleep state output unit 23 shares the sleep state acquired in step S13 with the service proposal application 30. Through the above steps, the service output system 1 outputs the sleep of the user state.

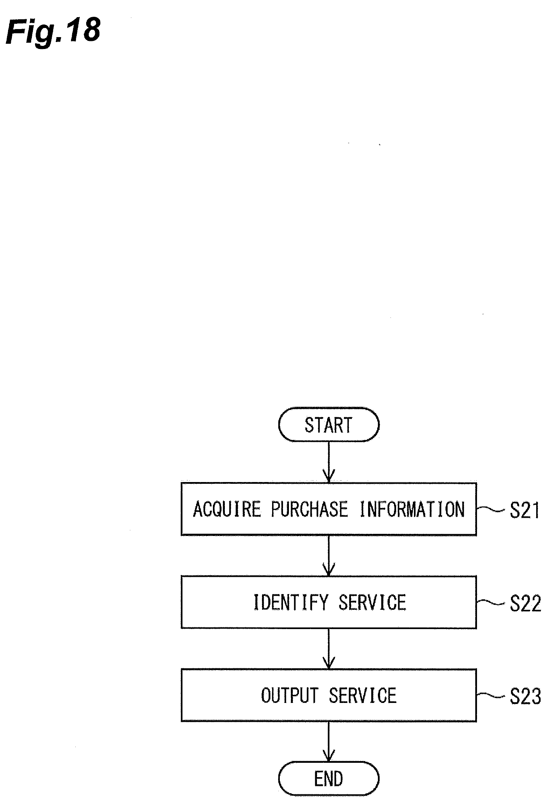

FIG. 18 is a flowchart showing an example of the operation of the service output system 1 until outputting a service. Before the service output system 1 executes the operation shown in FIG. 18, the customer information server 43 has generated purchase information from the order information received from the individual customer information server 42 or the user terminal 2. The customer information server 43 stores the generated purchase information. The customer information server 43 transmits the generated purchase information to the user terminal 2.

The purchase information acquisition unit 52 receives and acquires purchase information from the customer information server 43 (step S21). In step S21, the purchase information acquisition unit 52 transmits the acquired purchase information to the analysis device 3.

The service specifying unit 61 specifies a service recommended to the user (step S22). In step S22, the service specifying unit 61 receives and acquires the purchase information acquired in step S21. In step S22, the service specifying unit 61 receives and acquires the sleep state acquired in step S13. The service specifying unit 61 specifies a service recommended to the user from the purchase information acquired in step S21 and the sleep state acquired in step S13.

In step S22, the service specifying unit 61 acquires the preference of the user from the purchase information, for example. The service specifying unit 61 specifies provision of an advertisement of a product along the acquired preference as a service. When the acquired sleep score is equal to or less than a predetermined value, for example, the service specifying unit 61 specifies provision of an advertisement of a product capable of making sleep good as a service.

Next, the service output unit 62 outputs the service specified in step S22 toward the user (step S23). In step S23, the service output unit 62 transmits an e-mail indicating the specified service to the user terminal 2 as an output. Through the above steps, the service output system 1 outputs a service recommended to the user toward the user.

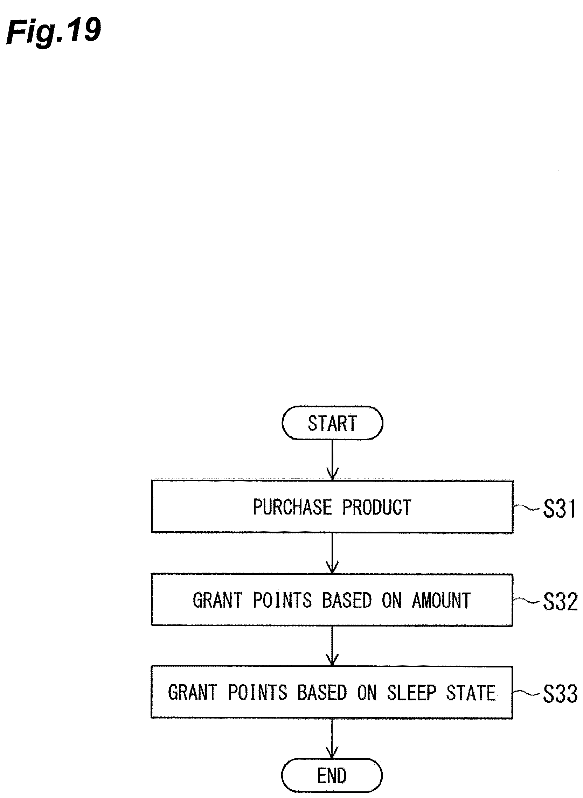

FIG. 19 is a flowchart showing an example of the operation of the service output system 1 until assigning points to the user. First, the user purchases a product (step S31). In step S31, the user purchases a product by, for example, the store terminal 41 or the user terminal 2.

When the user purchases a product by the store terminal 41 in step S31, the store terminal 41 transmits order information of the user to the main body unit 100 together with the identification information of the user. When the user purchases a product by the user terminal 2 in step S31, the user terminal 2 transmits order information of the user to the main body unit 100 together with the identification information of the user. When receiving the order information from the store terminal 41 or the user terminal 2, the main body unit 100 provides the product indicated by the order information to the user identified by the identification information included in the received order information.

When the store terminal 41 transmits the order information to the main body unit 100, the store terminal 41 transmits the order information to the individual customer information server 42 of the store where the store terminal 41 is arranged. When the user terminal 2 transmits the order information to the main body unit 100, the user terminal 2 generates point information indicating points according to the amount of money of the product included in the order information.

The point management server 44 assigns points according to the amount of money of the product to the user (step S32). In step S32, the point management server 44 receives point information from the individual customer information server 42 or the user terminal 2. The point management server 44 assigns points indicated by the received point information, that is, points according to the amount of money of the product, to the user. The point management server 44 stores held points held by the user.

Next, the point assigning unit 53 assigns points according to the sleep state to the user (step S33). In step S33, the point assigning unit 53 acquires the sleep state shared by the sleep state output unit 23 in step S16. The point assigning unit 53 assigns, for example, points of a value equal to the sleep score calculated in step S16 to the user.

The point management server 44 receives and acquires point information from the point assigning unit 53. The point management server 44 assigns points indicated by the acquired point information to the user. As a result, the point assigning unit 53 assigns points according to the sleep state to the user. Through the above steps, the service output system 1 assigns points to the user.

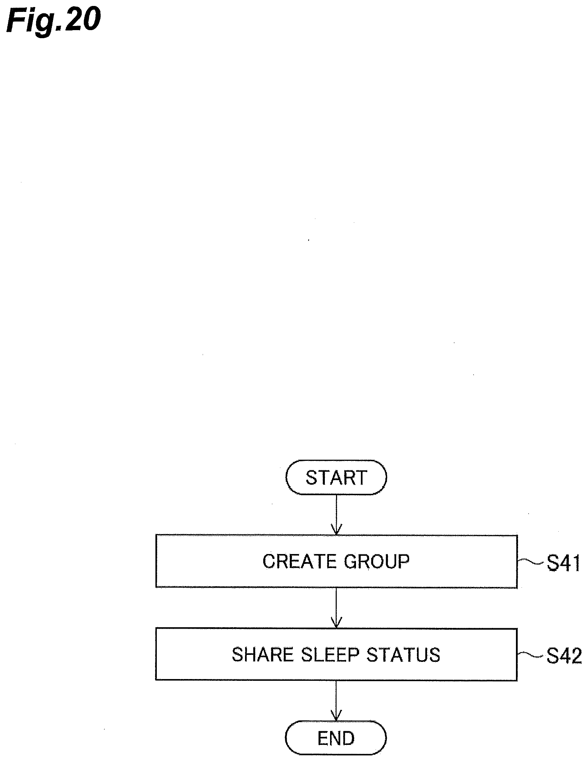

FIG. 20 is a flowchart showing an example of the operation of the service output system 1 until sharing a sleep state. The group configuration unit 28 generates a group composed of a plurality of users (step S41). In step S41, the group configuration unit 28 generates a group composed of a plurality of users to whom identification information has been assigned in step S1. In step S41, the group configuration unit 28 generates a group composed of a plurality of users arbitrarily determined by at least one user.

The sharing unit 29 shares the sleep of the user state (step S42). In step S42, the sharing unit 29 outputs the sleep of the user state to another user belonging to the group including the user. The sharing unit 29 outputs the sleep state of one user belonging to the group generated in step S41 to another user belonging to the group. Through the above steps, the service output system 1 shares the sleep of the user state.

The example of the steps of the operation of the service output system 1 has been described above. However, the content and order of the steps of the operation of the service output system 1 are not limited to the aforementioned example and can be changed as appropriate.

The operational effects of the service output system 1 will be described. In the service output system 1, purchase information indicating a product purchased by the user is acquired. Since the user often purchases a product based on their own preference, the preference of the user tends to be reflected in the purchase information. In the service output system 1, from the purchase information in which the preference of the user is reflected and the sleep state, the service specifying unit 61 specifies a service recommended to the user, and the service output unit 62 outputs the service toward the user. Therefore, by acquiring the sleep state, in addition to considering the actual situation of the sleep of the user, a service according to the preference of the user can be proposed. In the service output system 1, as the life improvement content, for example, information indicating an exercise for making sleep good can be provided toward the user. Therefore, the life of the user can be improved.

In the present embodiment, the sleep analysis application 20 and the service proposal application 30 can share information with each other. The service proposal application 30 assigns identification information identifying a user, and acquires purchase information of the user identified by the assigned identification information. The sleep analysis application 20 acquires the sleep state of the user identified by the identification information using the identification information assigned by the service proposal application 30. Since the user can be managed by the common identification information in both applications, the user can be managed uniformly. Since the application exhibits its function only by the user inputting the identification information and the password linked to the identification information to the service proposal application 30, the input amount of information by the user can be reduced.

In the present embodiment, the life improvement content includes audio content. The service output system 1 comprises the reception unit 25 that receives a selection of audio content by the user from a plurality of types of audio content set in advance. The content providing unit 26 provides the audio content selected by the user in the reception unit 25 at at least one of the time of falling asleep and the time of awakening of the user. Thereby, the user can enjoy the audio content selected by themselves at at least one of the time of falling asleep and the time of awakening. For example, when the user selects audio content suitable for falling asleep as the audio content provided at the time of falling asleep, the user can smoothly fall asleep by the audio content. For example, when the user selects audio content suitable for awakening as the audio content provided at the time of awakening, the user becomes easy to awaken by the audio content provided at the time of awakening.

In the present embodiment, the content providing unit 26 provides audio content according to the sleep state at the time of falling asleep and the time of awakening of the user. Since the audio content according to the sleep state is provided to the user, it is possible to make it easy for the user to fall asleep and awaken.

In the present embodiment, the content providing unit 26 provides advice according to the sleep of the user state. Since the user can receive advice according to their own sleep state, the user can make their own sleep good based on the advice.

In the present embodiment, the content providing unit 26 provides information prompting behavior modification according to the sleep of the user state. For example, when the sleep state of the user is not good, it is possible to prompt behavior modification of the user so as to consult a sleep specialist. Therefore, by prompting a change in usual behavior to the user, such as creating an incentive to consult a specialist for the user, the life of the user can be made good.

In the present embodiment, the service output system 1 comprises the point assigning unit 53 that assigns points according to the sleep state to the user. For example, in a case where larger points are assigned to the user as the sleep of the user state is better, it can be a motivation for the user to strive to take sleep. Therefore, the sleep of the user can be made good.

In the present embodiment, the service output system 1 comprises the sleep state output unit 23 that outputs the sleep state toward the user, and the device control unit 27 that controls the operation of the device 12 constituting an environment around the user from the sleep state. Since the sleep state is output to the user, the user can grasp their own sleep state. Since the operation of the device 12 such as an air conditioner arranged around the user can be controlled according to the sleep of the user state, the sleep of the user environment can be made comfortable according to the sleep state.

In the present embodiment, the sleep state output unit 23 outputs, as the sleep state, the state of the autonomic nerve of the user and the health risk due to the abnormal respiratory state of the user. Thereby, the user can grasp the state of their own autonomic nerve and the health risk due to the abnormal respiratory state. Therefore, the user can grasp their own sleep state in more detail.

In the present embodiment, the service output system 1 comprises the sharing unit 29 that outputs the sleep state of the user toward another user different from the user. Thereby, since the sleep state of the user is output toward the other user, the other user can grasp the sleep state of the user. Since the other user can grasp the sleep state of the user, the other user can promptly perform a countermeasure for the sleep state of the user as necessary.

In the present embodiment, the service output system 1 comprises the recording unit 24 that records sounds emitted in the user and around the user during sleep of the user. In this case, the user can confirm, for example, their own snoring which is difficult to be aware of, and sounds emitted in the sleep environment during sleep. By listening to the sounds emitted from the user, the user can grasp the state of their own body during sleep. Furthermore, by listening to the sounds emitted around the user, the user can grasp their own sleep environment. In this way, the user can grasp their own sleep state by listening to the sounds recorded in the recording unit 24.

The embodiments of the service output system according to the present disclosure have been described above. However, the present disclosure is not limited to the aforementioned embodiments, and various modifications are possible without changing the gist described in the claims.

In the aforementioned embodiment, an example in which the analysis device 3 has the service specifying unit 61 and the service output unit 62 has been described. However, the service proposal application 30 may have the service specifying unit 61 and the service output unit 62. In this case, the analysis device 3 may not be provided. In this case, the service specifying unit 61 may specify a service recommended to the user from the purchase information acquired by the purchase information acquisition unit 52 and the sleep state acquired by the sleep state acquisition unit 22.

The customer information server 43 may store activity information regarding activity during awakening of the user. The activity information includes, for example, at least one of the heart rate, blood pressure, and calorie consumption of the user during awakening. The activity information may be acquired by, for example, an activity meter worn by the user. The activity meter may be, for example, transferred or lent to the user from the sleep consultation office.

For example, the service proposal application 30 acquires the activity information of the user from the activity meter worn by the user. The service proposal application 30 transmits the acquired activity information to the customer information server 43. The customer information server 43 stores the activity information received from the service proposal application 30 for each user. The activity information stored in the customer information server 43 may be used for a specialist to provide advice to the user at the sleep consultation office. In this case, advice can be given to the user by considering not only the sleep of the user state but also the activity information of the user during awakening. Therefore, effective advice for making the health condition of the user good can be given.

The customer information server 43 may store interview information regarding the sleep of the user. The interview information includes, for example, at least one of the type of bedding used when the user takes sleep, and trouble regarding sleep. For example, when the user visits the sleep consultation office, an interview by a specialist is performed, and the customer information server 43 may store the content of the interview as interview information. For example, interview information may be input via a terminal installed in the sleep consultation office, and the terminal may transmit the input interview information to the customer information server 43. The customer information server 43 may store the interview information received from the terminal of the sleep consultation office for each user.

The customer information server 43 may store physical information regarding the body of the user. The physical information includes, for example, the height, weight, skeleton, and body shape of the user. The physical information includes, for example, body pressure when the body of the user is placed on bedding. For example, when the user visits a store, physical measurement of the user is performed at the store, and the customer information server 43 may store the result of the physical measurement as physical information. For example, physical information may be input via the store terminal 41. The store terminal 41 may transmit the input physical information to the individual customer information server 42. The individual customer information server 42 may transmit the physical information received from the store terminal 41 to the customer information server 43. The customer information server 43 may store the physical information received from the individual customer information server 42 for each user.

The physical information stored in the customer information server 43 may be used, for example, to manufacture the bedding of the user. In this case, for example, custom-made bedding according to the characteristics of the body of the user can be manufactured.

In the aforementioned embodiment, an example in which the money calculator arranged in the store is capable of cooperating with the store terminal 41 by the POS system has been described. However, the type of the money calculator is not limited to that described in the aforementioned embodiment. When the money calculator is not capable of cooperating with the store terminal 41 by the POS system, the service output system 1 may include a conversion program for cooperating with the money calculator.

### Reference Signs List

1... Service output system, 2... User terminal, 3... Analysis device, 10... Mattress, 11... Sensor unit, 12... Device, 13... Analysis server, 14... Short-term data server, 15... Long-term data server, 16... Content server, 20... Sleep analysis application, 21... User specifying unit, 22... Sleep state acquisition unit, 23... Sleep state output unit, 24... Recording unit, 25... Reception unit, 26... Content providing unit, 27... Device control unit, 28... Group configuration unit, 29... Sharing unit, 30... Service proposal application, 41... Store terminal, 42... Individual customer information server, 43... Customer information server, 44... Point management server, 45... Store information server, 51... Identification information assigning unit, 52... Purchase information acquisition unit, 53... Point assigning unit, 61... Service specifying unit, 62... Service output unit, 100... Main body unit, P1... Authentication screen, P2... Sleep state output screen, P3... Audio record display screen, P3a... Reproduction button, P4... Alarm setting screen, P5... Alarm content setting screen, P6... Talent voice setting screen, P7... Sleep sharing result screen, P8... Content selection screen, P9... Video content screen, P11... Identification information input unit, P12... Password input unit, P13... Determination unit, P14, Q10... Menu bar, P15... Alarm selection unit, P16... Sleep record selection unit, P17... Content selection unit, P21... Action record display unit, P22... Radar chart display unit, P23... Sleep graph display unit, P24... Audio record display unit, P25, P33... Sleep score display unit, P26... Advice display unit, P27... Specialist consultation unit, P28... Consultation office display screen, P29... Heart rate/respiration display unit, P30... Autonomic nerve display unit, P30a, P31a... Point, P31... Health risk display unit, P32... Sleep outpatient search unit, P34... Sleep time display unit, P35... Sleep stage display unit, P36... Sleep calendar display unit, P37... Average data display unit, P38... Sleep sharing screen instruction unit, P41... Target bedtime display unit, P42... Target sleep time display unit, P43... Reproduction time display unit, P44... Alarm setting unit, P45... Talent voice setting unit, P46... Alarm instruction unit, P47... Audio record instruction unit, P48... Mattress cooperation instruction unit, P49... Sleep sharing instruction unit, P71... First content selection unit, P72... Second content selection unit, P73... Third content selection unit, Q1... Member information display screen, Q11... Topics instruction unit, Q12... Store instruction unit, Q13... Shopping instruction unit, Q14... Service instruction unit, Q15... Member information instruction unit, Q21... Point history instruction unit, Q22... Purchase history instruction unit, Q23... Service history instruction unit, Q24... Basic information change instruction unit.

## Claims

1. A service output system comprising:
an identification information assigning unit that assigns identification information identifying a user for each user;
a user specifying unit that specifies the user identified by the identification information;
a sleep state acquisition unit that acquires a sleep state of the user;
a purchase information acquisition unit that acquires purchase information indicating a product purchased by the user;
a service specifying unit that specifies a service recommended to the user from the purchase information and the sleep state;
a service output unit that outputs the service toward the user; and
a content providing unit that provides life improvement content improving a life of the user.

2. The service output system according to claim 1,
wherein the life improvement content includes audio content, further comprising
a reception unit that receives a selection of audio content by the user from a plurality of types of audio content set in advance, and
wherein the content providing unit provides the audio content selected by the user in the reception unit at at least one of a time of falling asleep and a time of awakening of the user.

3. The service output system according to claim 1 or 2,
wherein the life improvement content includes audio content, and
wherein the content providing unit provides the audio content according to the sleep state at at least one of a time of falling asleep and a time of awakening of the user.

4. The service output system according to claim 1 or 2,
wherein the content providing unit provides advice according to the sleep state of the user.

5. The service output system according to claim 1 or 2,
wherein the content providing unit provides information prompting behavior modification according to the sleep state of the user.

6. The service output system according to claim 1 or 2, further comprising
a point assigning unit that assigns points according to the sleep state to the user.

7. The service output system according to claim 1 or 2, further comprising:
a sleep state output unit that outputs the sleep state toward the user; and
a device control unit that controls an operation of a device constituting an environment around the user from the sleep state.

8. The service output system according to claim 7,
wherein the sleep state output unit outputs, as the sleep state, a state of an autonomic nerve of the user and a health risk due to an abnormal respiratory state of the user.

9. The service output system according to claim 1 or 2, further comprising
a sharing unit that outputs the sleep state of the user toward another user different from the user.

10. The service output system according to claim 1 or 2, further comprising
a recording unit that records sounds emitted in the user and around the user during sleep of the user.
